# EUROPEAN PATENT SPECIFICATION

(11) **EP 0 722 431 B1**
(45) Date of publication and mention of the grant of the patent: **23.05.2001**
(21) Application number: 95927612.2
(22) Date of filing: 10.08.1995
(51) Int. Cl.: C07C 45/28, C07C 49/258, C07C 403/14, C07C 403/16, C07D 307/83, C07D 303/32, A61K 31/12

(54) **OXIDIZED CAROTENOIDS, RETINOIDS AND RELATED CONJUGATED POLYENES, AND DERIVED FRACTIONS AND COMPOUNDS USEFUL AS CELL-DIFFERENTIATION INDUCERS, CYTOSTATIC AGENTS, AND ANTI-TUMOR AGENTS**
WEITGEHEND OXIDIERTE CAROTENOIDE, RETINOIDE UND VERWANDTE CONJUGIERTE POLYENE, UND ABGELEITETE FRAKTIONEN, UND VERBINDUNGEN VERWENDBAR ALS ZELL-DIFFERENZIERUNGS-INDUKTOREN, CYTOSTATIKA UND ANTITUMOR MITTEL
CAROTENOIDES, RETINOIDES ET POLYENES, CONJUGUES APPARENTES OXYDES, AINSI QUE FRACTIONS ET COMPOSES DERIVES UTILES COMME INDUCTEURS DE DIFFERENCIATION CELLULAIRE, AGENTS CYTOSTATIQUES, ET AGENTS ANTITUMORAUX

(30) Priority: 10.08.1994 US 288315
(43) Date of publication of application: 24.07.1996
(73) Proprietor: NATIONAL RESEARCH COUNCIL OF CANADA, Ottawa Ontario K1A OR6 (CA)
(72) Inventor: BURTON, Graham, Orleans, Ontario K1W 1C2 (CA); DAROSZEWSKI, Janusz, Gloucester, Ontario K1J 6A8 (CA); PHIPPS, Jenny, Ottawa, Ontario K2C 3E2 (CA); ARYA, Prabhat, Gatineau, Quebec J8V 2R2 (CA)
(74) Representative: Perry, Robert Edward
(86) International application number: PCT/CA95/00484
(87) International publication number: WO 96/05160

(56) References cited:
- TETRAHEDRON (TETRAB,00404020);89; VOL.45 (24); PP.7679-94, ORTHO PHARM. CORP.;R. W. JOHNSON PHARM. RES. INST.; RARITAN; 08869-0602; NJ; USA (US), OYLER A R ET AL 'Characterization of autoxidation products of retinoic acid' cited in the application
- TETRAHEDRON LETT. (TELEAY,00404039);91; VOL.32 (33); PP.4203-6, UNIV. ST. ANDREWS;CHEM. DEP.; ST. ANDREWS/FIFE; KY16 9ST; UK (GB), MORDI R C ET AL 'Exploratory study of.beta.-carotene autoxidation' cited in the application
- TETRAHEDRON (TETRAB,00404020);93; VOL.49 (4); PP.911-28, UNIV. ST. ANDREWS;DEP. CHEM.; ST. ANDREWS/FIFE; KY16 9ST; UK (GB), MORDI R C ET AL 'Oxidative degradation of.beta.-carotene and.beta.-apo-8'-carotenal' cited in the application
- J. AGRIC. FOOD CHEM. (JAFCAU,00218561);90; VOL.38 (5); PP.1238-42, UNIV. SCI. TECH. LANGUEDOC;CENT. GENIE TECHNOL. ALIMENT.; MONTPELLIER; 34060; FR. (FR), KANASAWUD P ET AL 'Mechanism of formation of volatile compounds by thermal degradation of carotenoids in aqueous medium. 2. Lycopene degradation'

## Description

This invention relates to the use of oxidized carotenoids, retinoids and related conjugated polyenes and some derivatives thereof having cell-differentiation-inducing, cytostatic and anti-tumor properties, as chemotherapeutic and chemopreventive agents, and more particularly, to the use of such derivatives obtained by extensive oxidation of carotenoids, retinoids and related conjugated polyenes and subsequent processing.

### BACKGROUND OF THE INVENTION

Carotenoids and retinoids are naturally occurring substances which contain extensively conjugated polyene chains. Carotenoids have the most extensively conjugated systems of carbon-carbon double bonds which give rise to their many varied and brilliant colors. Many carotenoids and retinoids, which are naturally occurring substances, are biologically active. For example, certain hydrocarbon members of the carotenoid family (most notably, β-carotene, or pro-vitamin A, one of the most abundant carotenoids in food) are sources of retinol (one form of vitamin A); carotenoids protect plants from photosensitized oxidative damage, probably by deactivating singlet oxygen; epidemiological evidence indicates that carotenoid intake correlates inversely with the incidence of some types of cancer (Peto et al, Nature, 1981, 290, 201-208). Carotenoids and retinoids have been shown to retard the development of some experimentally induced animal tumors (N. I. Krinsky. Actions of Carotenoids in Biological Systems, Annu. Rev. Nutr, 13, 561-587 (1993); Matthews-Roth, Curr. Top. Nutr. Dis. [New Prot. Roles Select. Nutr.], 1989, 22, 17-38; Pure Appl. Chem., 1985, 57, 717-722); a number of dietary intervention studies are being carried out to try to determine the efficacy of supplemental B-carotene as a non-toxic, dietary anti carcinogen that can effectively decrease cancer mortality and most recently the possibility has begun to be examined that β-carotene may be associated with decreased incidence of coronary heart disease; recent clinical data with the use of related compounds (retinoids - retinoic acid, retinol and retinamides) have demonstrated a role in anti-cancer therapy, both as a therapeutic and a preventive agent (cancers of the skin, head and neck, lung and bladder, acute promyelocytic leukemia, leukoplakia and myelodysplastic syndromes; D.L. Hill and C.J. Grubs, Retinoids and Cancer Prevention, Annu. Rev. Nutr. 1992, 12, 161-181); and finally, β-carotene has antioxidant properties at the low oxygen pressures found in tissues (Burton and Ingold, β-Carotene: an unusual type of lipid antioxidant, Science, 1984, 224, 569-573).

Carotenoids, retinoids and related conjugated polyenes are reactive towards molecular oxygen (O₂) and may therefore be oxidatively degraded in foodstuffs during storage, even at reduced temperatures. Carotenoids are more reactive than retinoids towards oxygen because of their larger, more extensively conjugated system of double bonds. The products of such oxidative degradation of carotenoids retinoids, and related conjugated polyenes and their potential physiological activities have, nevertheless, received remarkably little attention, with the exception of vitamin A, which is obtained as a product of the biological oxidation of β-carotene.

Mordi et al, Exploratory study of β-carotene Autoxidation, published in Tetrahedron Letters, 1991, 32 (33), 4203-4206, examined the products formed during the self-initiated autoxidation of β-carotene. The paper concludes that the main products identified in the early stages of β-carotene autoxidation are epoxides, β-ionone, β-apo-13-carotenone, retinal, and related carbonyl compounds; in the final mixture, short chain carbonyl compounds predominate.

Another paper by Mordi et al, "Oxidative Degradation of β-carotene and β-Apo-8'-carotenal", published in Tetrahedron Vol. 49, No. 4, pp. 911-928, January 22, 1993, shows that self-initiated oxidation of β-carotene with molecular oxygen produces epoxides, dihydrofurans, carbonyl compounds, carbon dioxide, traces of alcohols, and some other compounds. The paper, co-authored by one of the present inventors, also makes a mention of some polymeric/oligomeric material which frequently deposited out of solution, particularly in the later stages of β-carotene oxidation. The properties of the polymer/oligomer are not disclosed in the paper.

This patent application results from the development of our idea that the biological activity of carotenoids derives not from the carotenoids themselves but instead from one or more of their oxidation products generated in vivo. Retinoids are also included because of their ability to oxidize, although not as readily as carotenoids. The biological activity of oxidized retinoids is distinct from the known activity of the retinoids themselves.

### SUMMARY OF THE INVENTION

It has been found in a first phase of the research leading to the invention that mixtures obtained by oxidation of β-carotene, canthaxanthin or retinoic acid with oxygen, O₂, under conditions in which at least some of the substrate reacts, on a molecular basis, with a several-fold greater amount of oxygen (a process hereinafter referred to as extensive oxidation) are able, in a cell culture, to induce cancer-derived and virally-transformed mammalian cells to proliferate less rapidly in a manner that is non-toxic to normal cells. Furthermore, it has been. determined in several of the treated cell lines that cell differentiation occurs, i.e., the cancer-like cells eventually acquire many of the characteristics of normal cells. It has also been found that the mixture of material obtained from extensively oxidized B-carotene is able to retard or arrest, in a non-toxic manner, the growth of tumors in mice.

Therefore, the present invention provides the use of a product for the manufacture of a medicament for use in treating a tumour or skin cell proliferation in a human or animal, wherein the product is a mixture containing more than one oxidized compound, obtainable by the process of oxidation of a carotenoid compound with oxygen, such that 6 to 8 molecules of oxygen react per molecule of the carotenoid compound.

It is proposed to oxidize carotenoids, retinoids or related conjugated polyenes with oxygen in conditions effective to obtain a mixture containing an oligomeric or polymeric component.

The oxidation can be carried out with oxygen and a carotenoid, retinoid or conjugated polyene, either in the solid state or dissolved in an organic solvent.

Based on laboratory tests as described below, both the oxidized mixtures (and the oligomeric/polymeric component) obtained upon oxidation of β-carotene, canthaxanthin, lycopene, retinoic acid and partially oxidized mixtures thereof are believed to be effective as anti-proliferative and anti-tumor agents and differentiation inducers.

The structural formulae of the polymeric ingredients were not determined in the first phase of the research. There is evidence that the polymers formed upon oxidation of β-carotene, canthaxanthin or retinoic acid have a polyperoxide structure and contain acidic groups.

To address the question of the substances of the present invention occurring in nature, it will be appreciated that formation of polymeric components during oxidation is likely to be retarded in nature as non-isolated carotenoids and retinoids are protected against oxidation by antioxidants such as vitamin E. However, a polar material (which polymerized upon isolation), obtained by oxidizing β-carotene in the presence of vitamin E in solution, was as active in in vitro cell culture tests as the polymeric material obtained in the uninhibited oxidation.

The molecular weight of much of the polymeric component of the mixture of the invention is relatively low compared to the molecular weights of carotenoids and retinoids. For this reason, the material can be termed an oligomer as well as a polymer. For the purpose of the instant specification, the term polymer, or polymeric, will be used to define the material.

It has been determined in a second phase of the research work leading to the present invention that the mixture of the invention, obtained by the extensive oxidation as defined above, can be fractionated according to molecular weight. The fractionation may be carried out for example by solvent precipitation or by gel permeation chromatography. Certain fractions thus obtained can be further fractionated (sub-fractionated) according to their polarity. It has been found that many of the fractions and sub-fractions thus obtained, and certain compounds contained therein, exhibit an anti-tumor, cytostatic and/or cell-differentiation activity comparable or higher than the respective activities of the mixture defined in the first phase of the research work.
A specific compound, 2-methyl-6-oxo-2,4-heptadienal was isolated and found to exhibit cytostatic and anti-tumor properties. The compound can be obtained by fractionation of the extensively oxidized mixture of the invention (oxidized β-carotene), or by a conventional synthetic method. This compound has been reported previously as a product obtained by the oxidation of trans-retinoic acid, Oyler et al, "Characterization of autoxidation products of retinoic acid" published in Tetrahedron Vol. 45, No. 25, pp. 7679-7694, 1989.

### BRIEF DESCRIPTION OF THE DRAWINGS:

In the drawings:
Fig. 1 shows a reverse phase High Pressure Liquid Chromatography (HPLC) separation/analysis of extensively oxidized β-carotene;
Fig. 2 illustrates the results of gel permeation chromatography (GPC) analysis of extensively oxidized β-carotene;
Fig. 3 represents Fourier Transformed Infrared (FTIR) spectrum of the oxidation mixture from extensively oxidized β-carotene;
Fig. 4 represents a proton Nuclear Magnetic Resonance (NMR) spectrum of the oxidation mixture of extensively oxidized β-carotene;
Fig. 5a represents the UV spectrum of the polymeric component of the oxidation mixture of extensively oxidized β-carotene (fraction 1);
Fig. 5b represents UV spectrum of β-carotene;
Figs. 6a-6d illustrate the results of GPC analysis of specific fractions of the polymeric mixture as follows:
   6a - fraction 1
   6b - fraction 2,
   6c - fraction 3, and
   6d - fraction 4;
Fig. 7 represents the FTIR spectrum of fraction 1 of the polymeric mixture;
Fig. 8 illustrates the FTIR spectrum of the material obtained by oxidation of solid β-carotene;
Fig. 9 illustrates the results of GPC analysis of the polymeric material obtained by oxidation of solid β-carotene;
Figs. 10a-10f (photographs) illustrate the effect of retinoic acid and extensively oxidized β-carotene on differentiation of ES cells as follows:

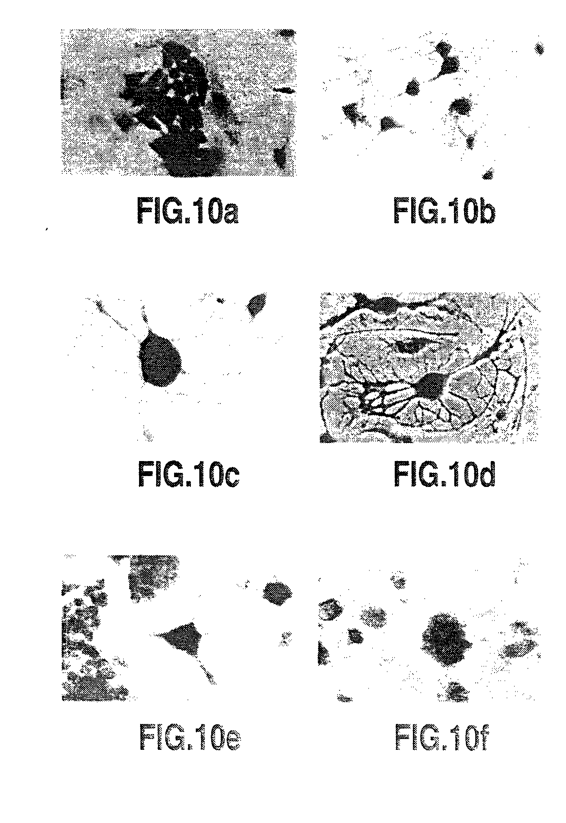

   Fig. 10a - ES cells, no inducer
   Fig. 10b - retinoic acid
   Fig. 10c - extensively oxidized β-carotene 3 *µ*M
   Fig. 10d - extensively oxidized β-carotene 7.5 *µ*M
   Fig. 10e - extensively oxidized β-carotene 15 *µ*M
   Fig. 10f - extensively oxidized β-carotene 30 *µ*M;

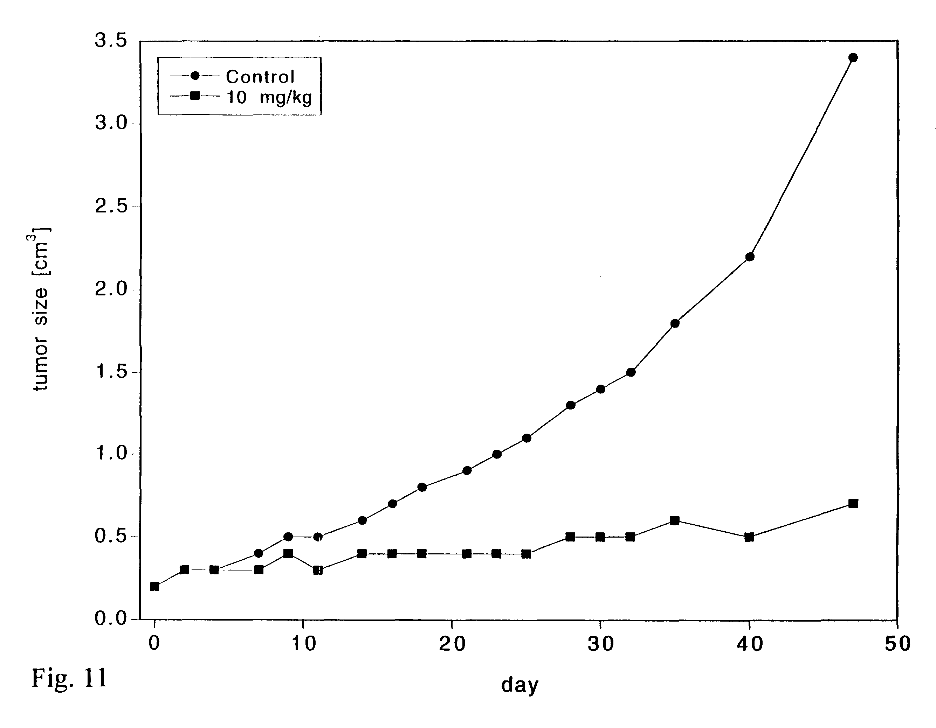

Fig. 11 illustrates the effect of extensively oxidized β-carotene on tumor growth at dose of 10 mg/kg injected on days 0,2,4,7,9,11,14,16 and 18;

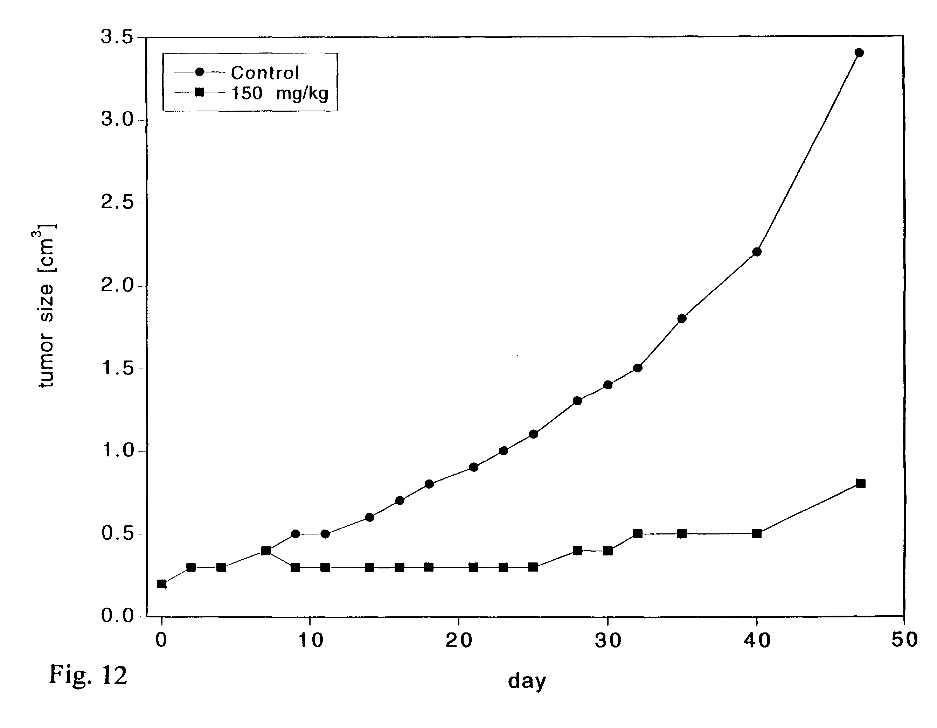

Fig. 12 illustrates the effect of extensively oxidized β-carotene on tumor growth at dose of 150 mg/kg injected on days 0,2,4,7,9,11,14,16 and 18;

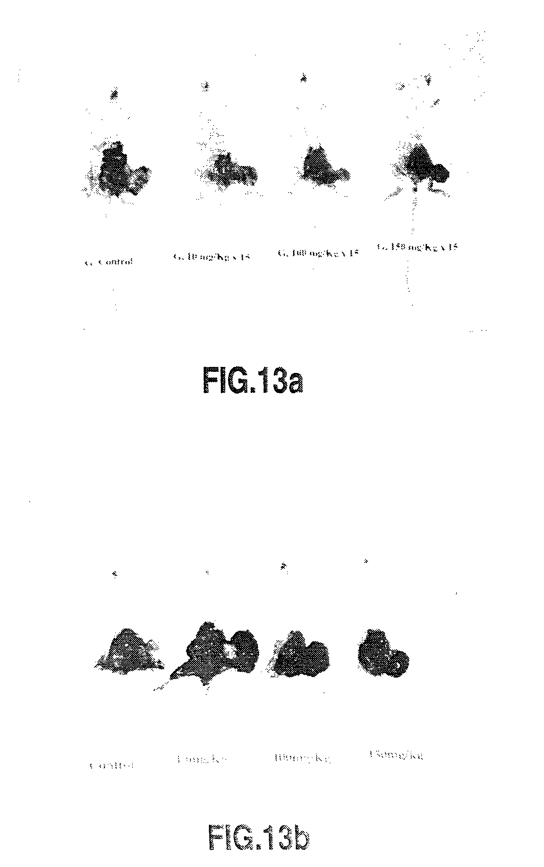

Fig. 13a illustrates the effect of extensively oxidized β-carotene on tumor size in sacrificed animals which had been treated with different doses of extensively oxidized β-carotene and the control (untreated animal);
Fig. 13b illustrates the effect of extensively oxidized β-carotene on tumor size in sacrificed animals which had been treated with different doses of extensively oxidized β-carotene and developed hemorrhaging around the tumors and the control (untreated animal);

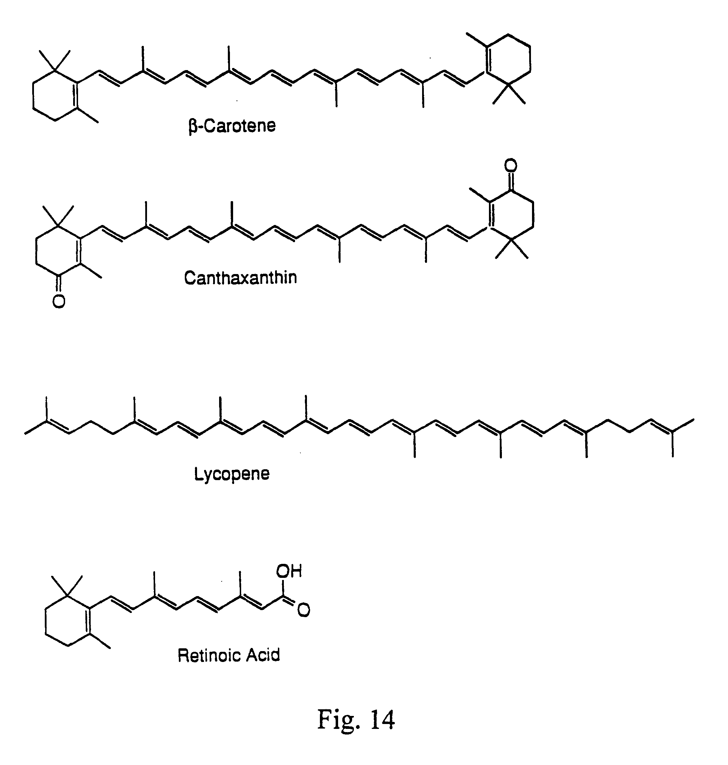

Fig. 14 represents, for comparison, the structural formulae of the main starting compounds for the purpose of this invention;

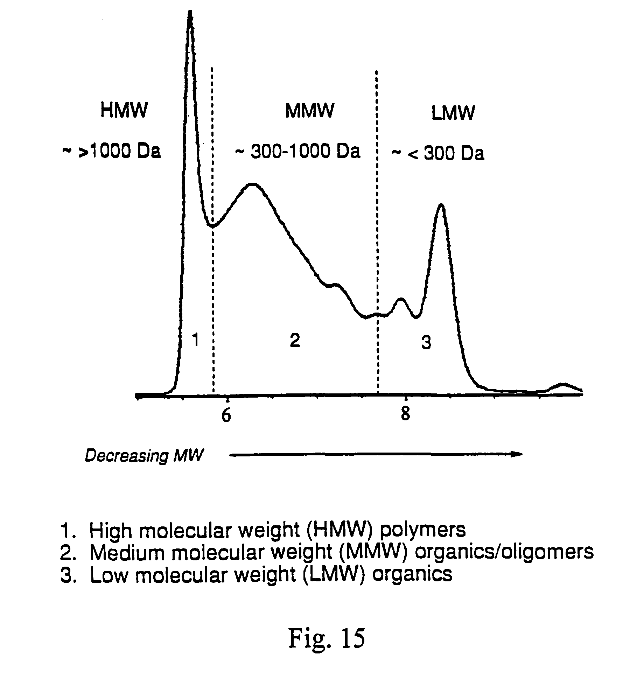

Fig. 15 illustrates the molecular weight split of the main fractions of the invention;

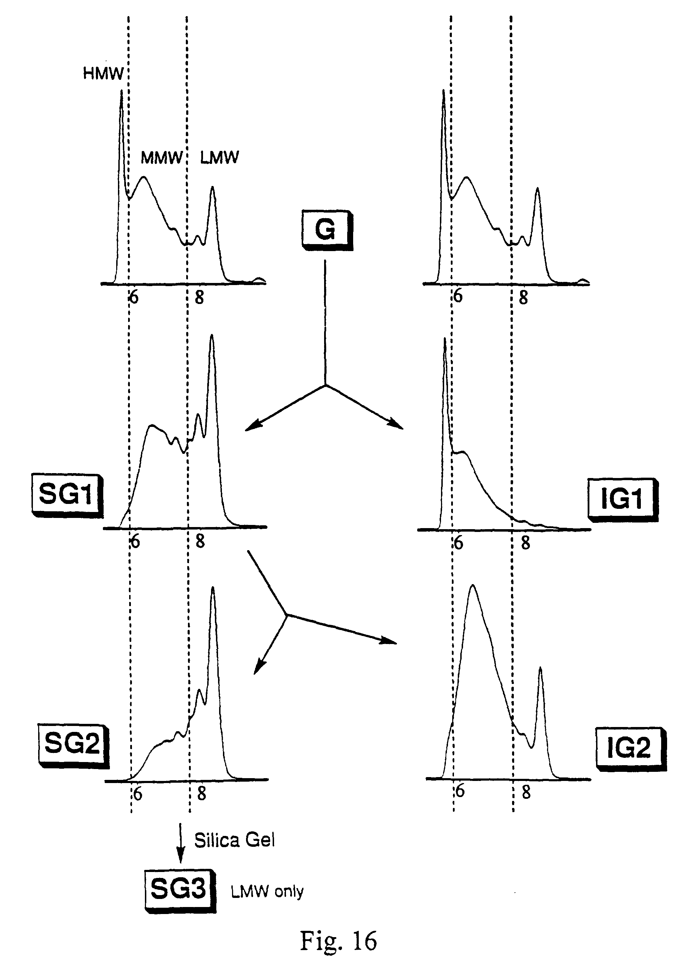

Fig. 16 illustrates schematically the three-level solvent mediated fractionation of the mixture;

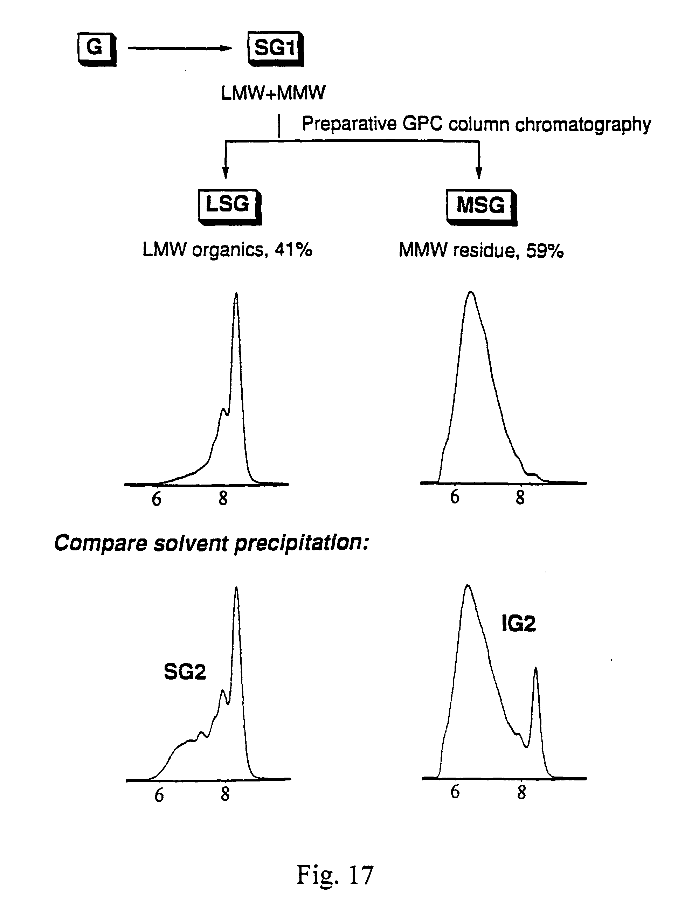

Fig. 17 illustrates GPC chromatogram of a preparative GPC separation of fraction SG1 into fractions LSG and MSG;

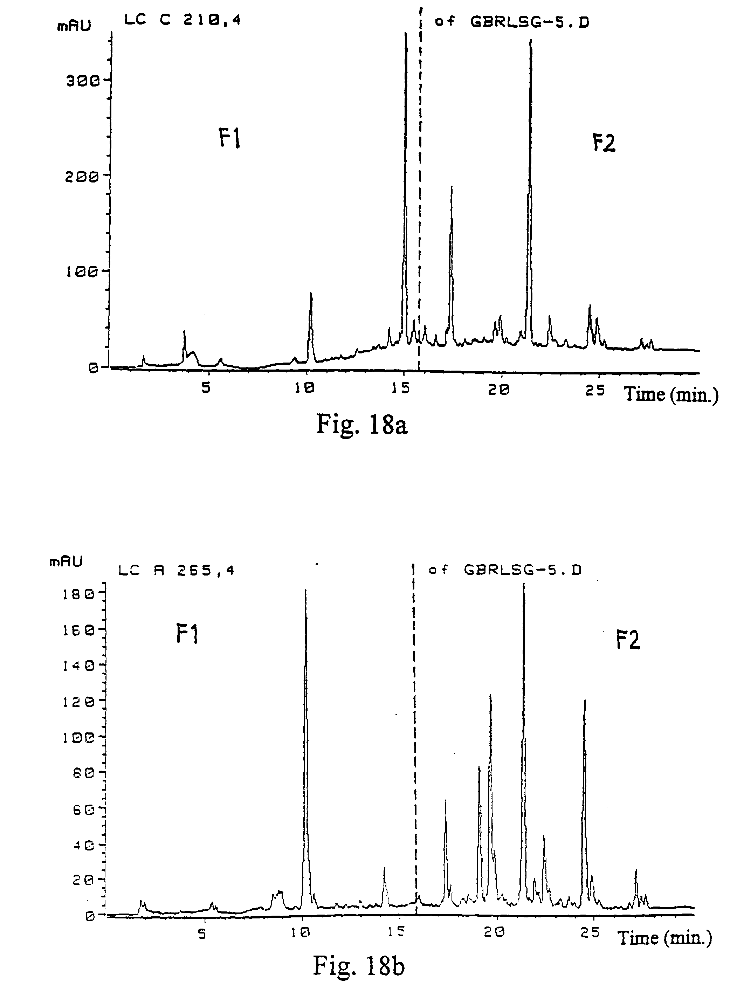

Figs. 18a and 18b illustrate HPLC chromatograms of fraction LSG, monitored at 219 and 265 nm respectively, and show how fraction LSG was split into fractions F1 and F2;

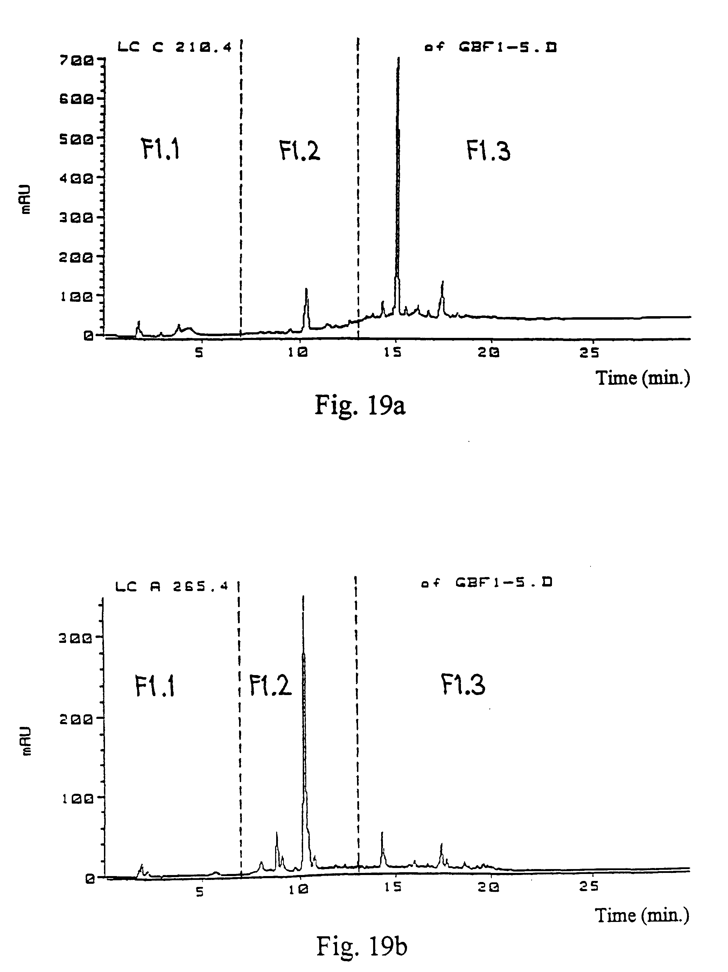

Figs. 19a and 19b illustrate HPLC chromatograms of fraction F1, monitored at 219 and 265 nm respectively, and show how fraction F1 was split into fractions F1.1 and F1.2 and F1.3;

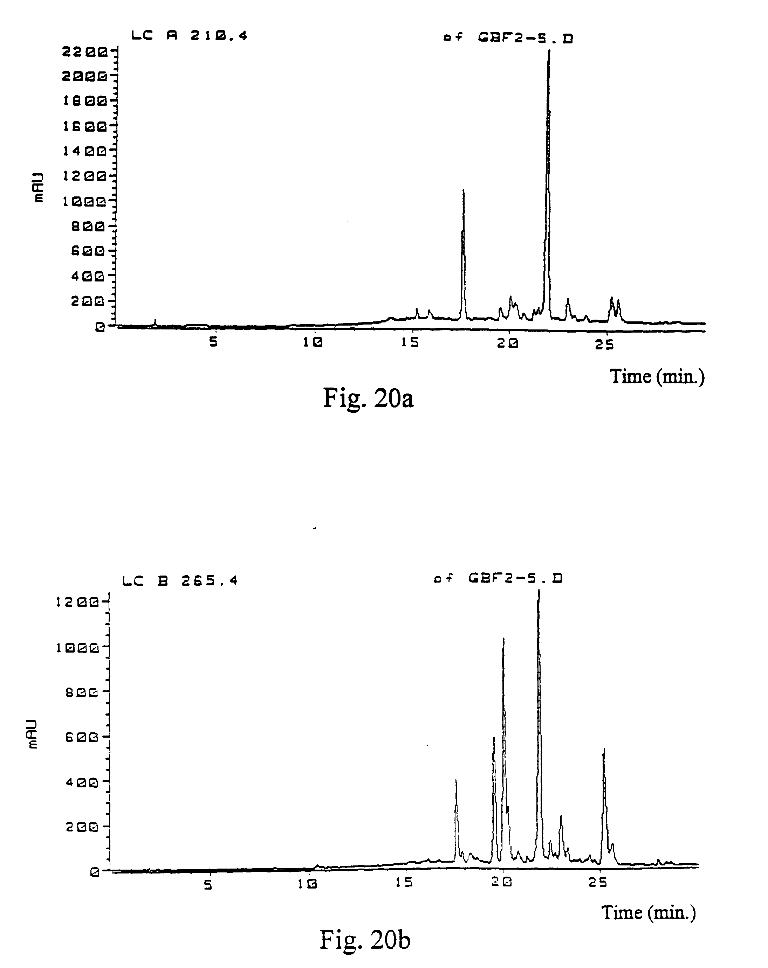

Figs. 20a and 20b show an HPLC chromatogram of fraction F2, monitored at 219 and 265 nm respectively;

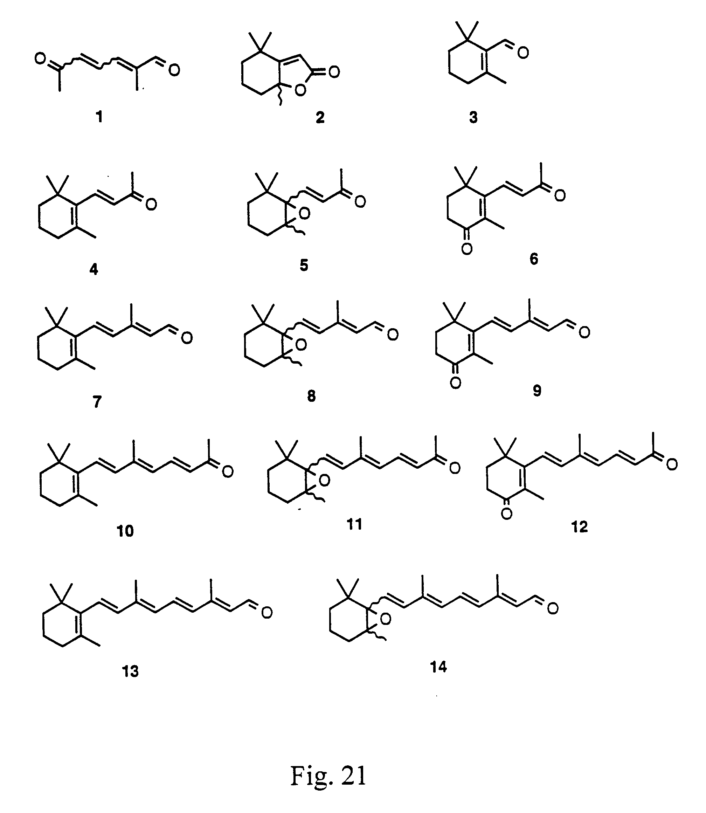

Fig. 21 illustrates the formulae of specific compounds identified the fractions of the invention;

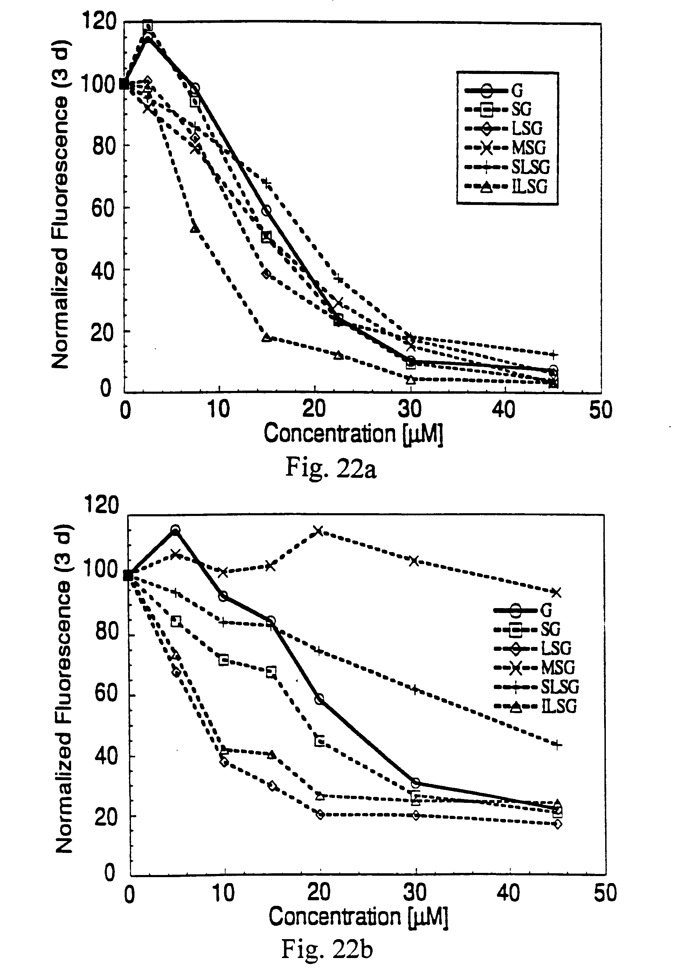

Fig. 22a shows cytostatic effect of certain fractions of the invention on a colon cancer cell line;
Fig. 22b shows cytostatic effect of certain fractions of the invention on a leukemic cell line;

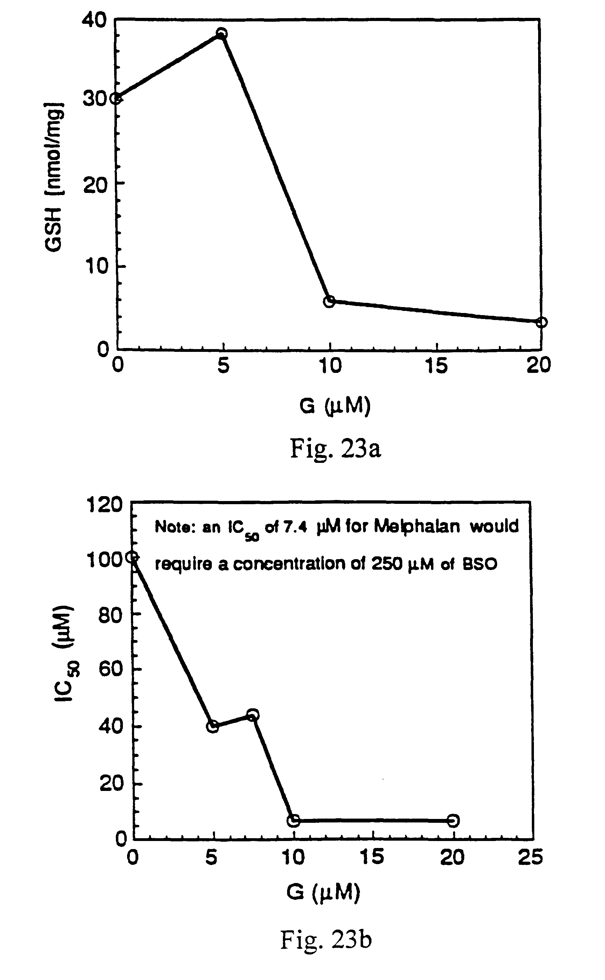

Fig. 23a illustrates the in vitro effect of β-car^{ox} on glutathione level in the DA-3 cell line;
Fig. 23b illustrates the in vitro effect of β-car^{ox} on IC₅₀ of Melphalan in the DA-3 cell line;

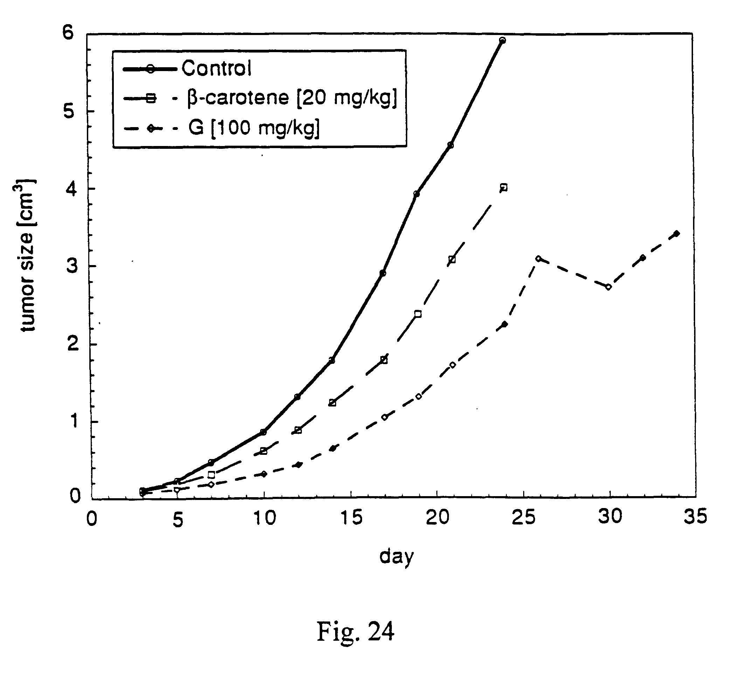

Fig. 24 shows antitumor properties of β-carotene and oxidized β-carotene in ovarian cancer; and

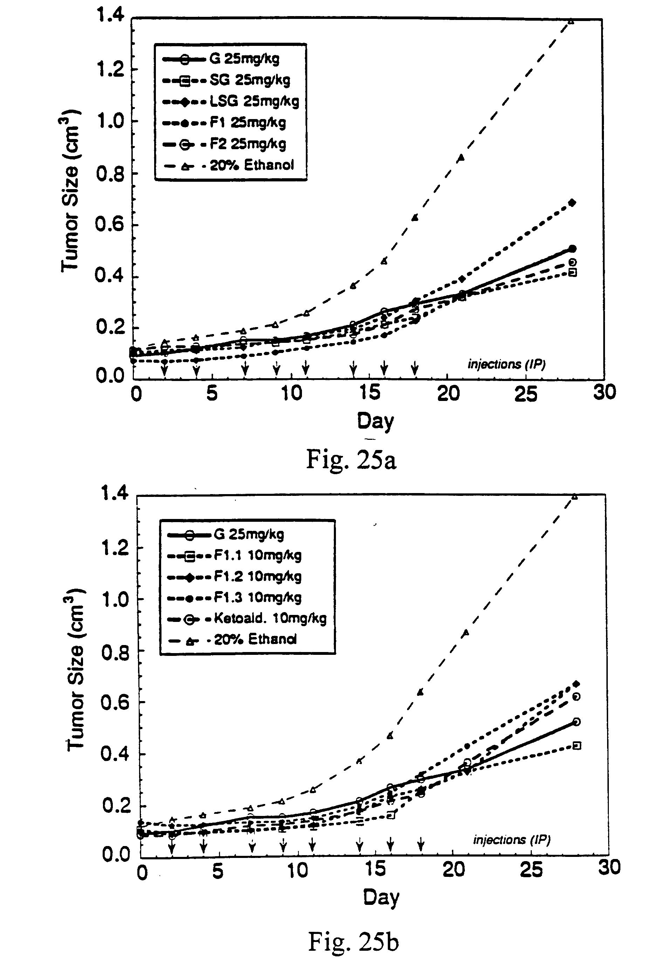

Figs. 25a and 25b show the inhibitory effect of various fractions of the invention on tumor growth in the mouse DA3 model.

### DETAILED DESCRIPTION OF THE INVENTION

β-Carotene, retinoic acid and related compounds have been identified as potential anti-cancer agents or even used as preventive and/or therapeutic agents in the treatment of different forms of cancer e.g. lung cancer and some forms of leukemia. The chemopreventive action of β-carotene has also received some attention in connection with the mode of action of vitamin A (retinoic acid), itself an oxidation product of β- carotene. Vitamin A has been shown to be capable of causing some types of cancer cells to at least partly revert from their proliferative, embryonic-like state to that resembling normal cells. However, the severe toxicity of vitamin A strictly limits its therapeutic applications.

The mechanism by which the carotenoids act is not yet understood. The actions of vitamin A itself and related retinoids which affect cell growth and differentiation appear to be mediated via the retinoid receptors located at the cell nucleus.

Regarding β-carotene and possibly other carotenoids, it has been widely believed but not proven that its anti-cancer effects derive somehow from the anti-oxidant properties of the intact molecule and not from its ability to form vitamin A.

Isolated β-carotene and other carotenoids readily undergo spontaneous oxidation by reaction with oxygen in air. Retinoids also are capable of undergoing spontaneous oxidation. However, it is appreciated from the outset that the presence of fewer conjugated olefinic bonds in retinoids diminishes the rate and extent of their spontaneous oxidation.

Spontaneous oxidation may cause carotenoids and retinoids to behave in vivo as intracellular pro-oxidants, acting as sources of biologically active radicals and/or radical-derived products. Free radicals and products of free radical oxidation have been recognized to act as secondary messengers playing a significant role in the signaling pathways of living cells. Indeed, although it has been recognized for a long time that the production of free radicals is an inevitable consequence of life in an aerobic environment and this was generally regarded as harmful to cells, more recently there has been a growing appreciation that free radicals, particularly oxy-radicals, play an important role in the maintenance, control and development of cells.

It is our discovery that the products of extensively oxidized carotenoids, retinoids and related conjugated polyenes and their structural analogs, possess non-vitamin A bioactivity. We have demonstrated this by extensively pre-oxidizing β-carotene, canthaxanthin and retinoic acid in vitro and testing the mixture of oxidation products for biological activity. It is important to recognize the distinction between the products of extensive oxidation, which are the basis of the present invention, from vitamin A which is the well-known product of the in vivo oxidative conversion of β-carotene and other vitamin A-yielding carotenoids.

In the second phase of the research work, a fractionation scheme was developed for the extensively oxidized β-carotene mixture, termed herein β-car^{ox}. The mixture was split into three fractions based on molecular weight (MW) distribution using a combination of solvent precipitation and gel permeation (size exclusion) chromatography (GPC). The biological assays indicate that most of the activity resides in the low and medium MW fractions. Therefore, attention has been focused on these two fractions, particularly the low MW fraction. More time is required to explore the more complex medium MW fraction.

The low MW fraction has been fractionated further using solvent precipitation to begin with but later using reverse phase high performance liquid chromatography (RP-HPLC). The point has been reached at which some fractions contain only one or just a few major compounds. Some of these compounds have been identified. One compound, an unsaturated ketoaldehyde, has been obtained by an independent route (synthesis) and has been found to have strong cytostatic and antitumor properties. This compound has not previously been identified as being formed from β-carotene but has been reported as a product of the autoxidation of RA. It appears that the cytostatic and antitumor properties of the unsaturated conjugated ketoaldehyde class of compounds has not been recognized before.

Many of the β-car^{ox} fractions have been assayed for cytostatic and differentiation-inducing activities. Several of the fractions have stronger cytostatic activity than β-car^{ox}. Induction of differentiation has been tested in several human cell lines. The strongest effects are seen with the IMR32 neuroblastoma line. The most striking result has been the observation that precursor cells in a single cell line can give rise to two different phenotypes (neurons and glial cells) when treated with different fractions. (Substances with such properties could find application in neural regeneration therapies for brain and spinal cord injuries.)

β-Car^{ox} antitumor activity has been demonstrated for a human ovarian cancer line grown as a xenograft in nude mice. Nude mice xenografts derived from human cell lines are regarded as closer reflections of human tumors than animal tumors derived from animal cell lines. Testing of many of the β-car^{ox} fractions using the in vivo model described in the first patent has shown several fractions have strong activity. It is noteworthy too that activity is achieved at concentrations which exhibit no overt toxicity in the animals.

At this time, after the second phase of the research work, we have determined that more than one compound (derived from the oxidized mixture as defined hereinabove) is active as an anti-cancer agent and that fractions which contain more than one compound are more active than single compounds.

Lycopene (see Fig. 14), a carotenoid found in tomatoes, also has been found to possess cytostatic activity after extensive oxidation. Lycopene lacks the two cyclohexyl rings present in β-carotene.

To validate the present invention, in vivo and in vitro biological tests were conducted, and methods of synthesis and analytical data on the oxidation mixtures of β-carotene, canthaxanthin and retinoic acid are set forth hereinbelow.

### EXPERIMENTAL

### FIRST PHASE OF RESEARCH.

### Chemistry

β-Carotene, canthaxanthin and retinoic acid were oxidized as described below:

### Example 1: Oxidation of β-carotene

A 20 mM solution of β-carotene (Fluka) in benzene saturated with oxygen was incubated in a shaker bath, in the dark, at 30°C under pure oxygen at atmospheric pressure. After 72 hours, when 6 to 8 molar equivalents of oxygen had been consumed, the solvent was evaporated to give a resin-like, yellow residue.

### Effect of solvent:

Oxidation of β-carotene in carbon tetrachloride yielded results essentially identical to those obtained in benzene.

### Quantity of vitamin A formed:

Neither retinol nor retinoic acid, both products of the in vivo, enzymatic oxidation of β-carotene, were detected in the oxidation mixture. Although retinal, which can be oxidized to retinoic acid, has been identified as a product it is present in too small amount to account for the biological activity of the oxidized β-carotene mixture. Furthermore, the biological activity of the oxidized mixture differs substantially from that of vitamin A, as will be described below.

### Polymeric Materials:

Very substantial amounts of polymeric substances are formed during the oxidation (see below). It is likely, by analogy with the oxidation reactions of other olefinic compounds, that the higher molecular weight substances correspond to polymers made up of oxidized β-carotene fragments. Various concentrations of β-carotene were tested to determine the dependence of polymerization upon the concentration of β-carotene in solution. 20 mM, 2 mM and 0.2 mM solutions of β-carotene in benzene, saturated with oxygen, were incubated under pure oxygen (760 mm Hg) at 30°C in the dark. The polymeric ingredient was the main product in all cases. Furthermore, the polymeric ingredient forms early in the oxidation of β-carotene.

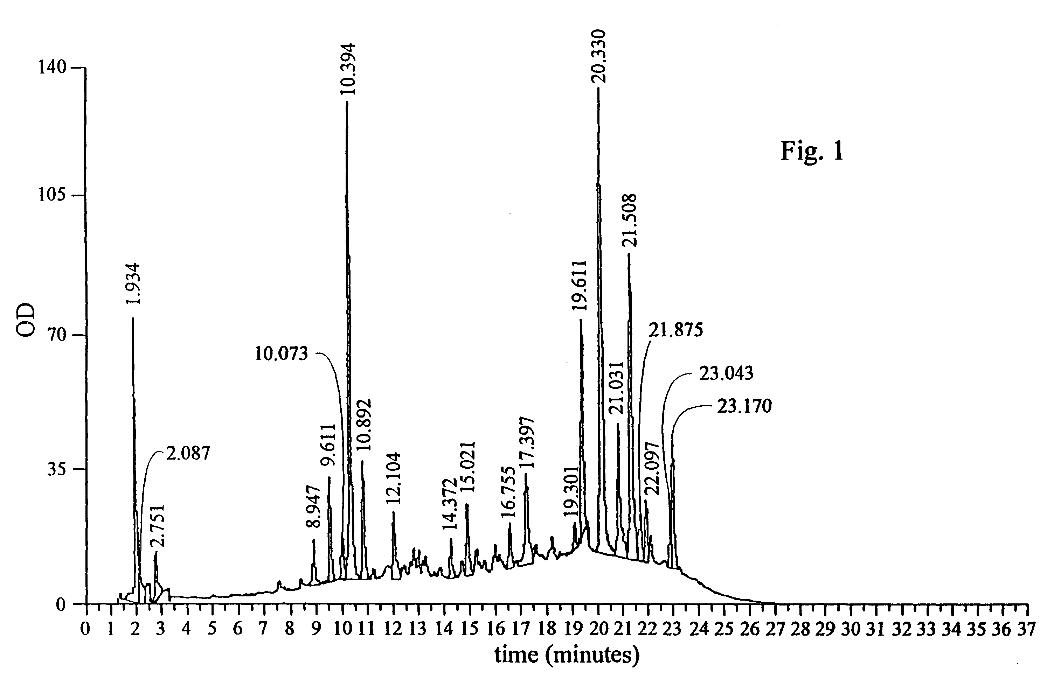

Fig. 1 presents a reversed phase High Performance Liquid Chromatography (HPLC) characterization of the oxidation mixture.

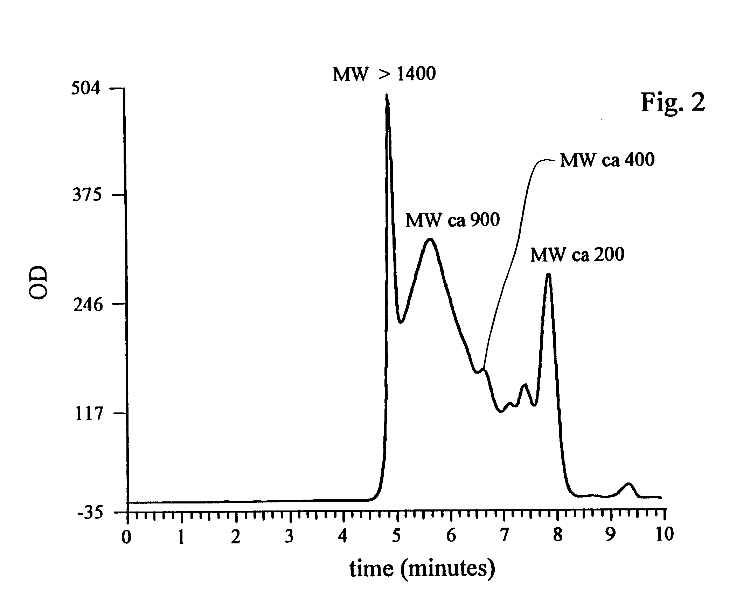

Fig. 2 presents a GPC characterization of the oxidation mixture showing, in arbitrary units, the composition of the oxidation mixture according to the molecular weight of the components. The molecular weight range is broadly distributed from about 600 to about 8000 Dalton with the maximum at about 900 Dalton (the sharp peak at 4.9 min. is an artifact, due to the nature of the GPC column, all the components of the mixture with molecular weight above ca. 1400 elute simultaneously).

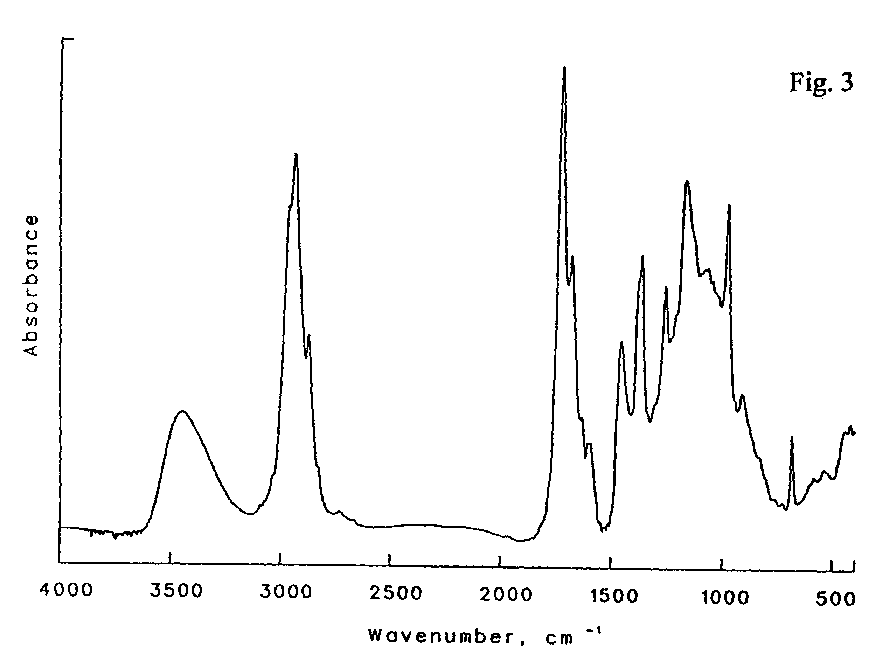

Fig. 3 is a Fourier Transformed Infrared (FTIR) spectrum of the oxidation mixture.

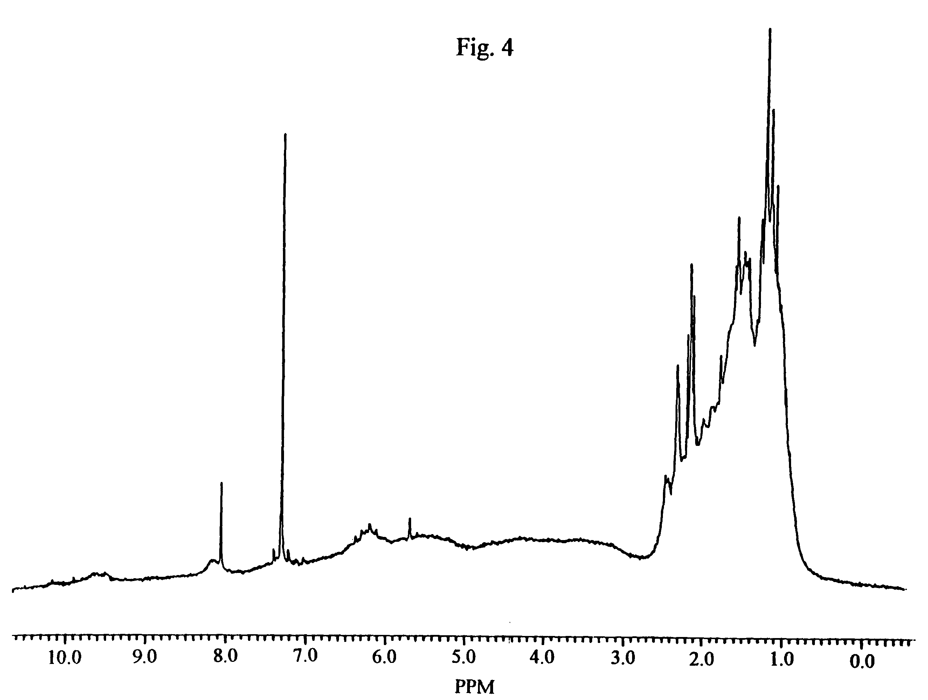

Fig. 4 is a proton Nuclear Magnetic Resonance (NMR) spectrum of the oxidation mixture.

### Partial Fractionation of the Mixture of Products from β-Car^{ox}:

An extensively oxidized mixture with no B-carotene remaining, obtained in solution, was fractionated by successive solvent precipitations. The fractions were characterized by gel permeation chromatography (GPC) and by elemental analysis. The results of elemental analysis and analysis of acid and peroxide content are shown in Table 1:

**Table 1.**

| **Characteristics of Various Fractions Obtained by Successive Solvent Precipitations of β-Car**^{**ox**}**.** | | | | |
|---|---|---|---|---|
| Fraction | 1 | 2 | 3 | 4 |
| Weight % | 37 | 17 | 14 | 32 |
| | | | | |
| Titratable acids [10⁻⁴ mol/g] | 4.3±0.3 | 4.3±0.2 | 6.1±0.3 | 6.2±0.1 |
| | | | | |
| Peroxides (iodometric) [10⁻⁴ mol/g] | 9.5±1.0 | 9.9±0.8 | 8.2±0.3 | 5.9±0.4 |
| | | | | |
| Peroxides (oxidation of Fe²⁺) [10⁻⁴ mol/g] | 8.3±0.2 | 8.9±0.2 | 8.8±0.9 | 5.5±0.1 |
| | | | | |

| Elemental Analysis: | | | | |
|---|---|---|---|---|
| C | 58.5 | 58.7 | 61.2 | 65.3 |
| H | 7.2 | 7.0 | 7.6 | 8.7 |
| O | 34.3 | 34.3 | 31.2 | 26.0 |
| Fractions were obtained in the following way: the crude mixture (1.2 g) was dissolved in tetrahydrofuran (THF) (5 ml) and hexane (15 ml) was added slowly while the solution was vortex-mixed. The sample was centrifuged (3000 rpm, 3 min.) and the oily residue was separated, washed once with a mixture of THF and hexane (5:15) and dried under vacuum to yield fraction 1. The remaining solution was combined with the liquid from the washing, evaporated and the residue dissolved in THF (3 ml). Precipitation with hexane (15 ml) followed by centrifugation and washing (THF/hexane, 3:15) gave fraction 2. As before, the remaining solution, combined with the wash, was evaporated and dissolved in benzene (3 ml). Precipitation with hexane (15 ml) followed by washing (benzene/hexane, 3:15) gave fraction 3. The supernatant material was labeled as fraction 4. | | | | |

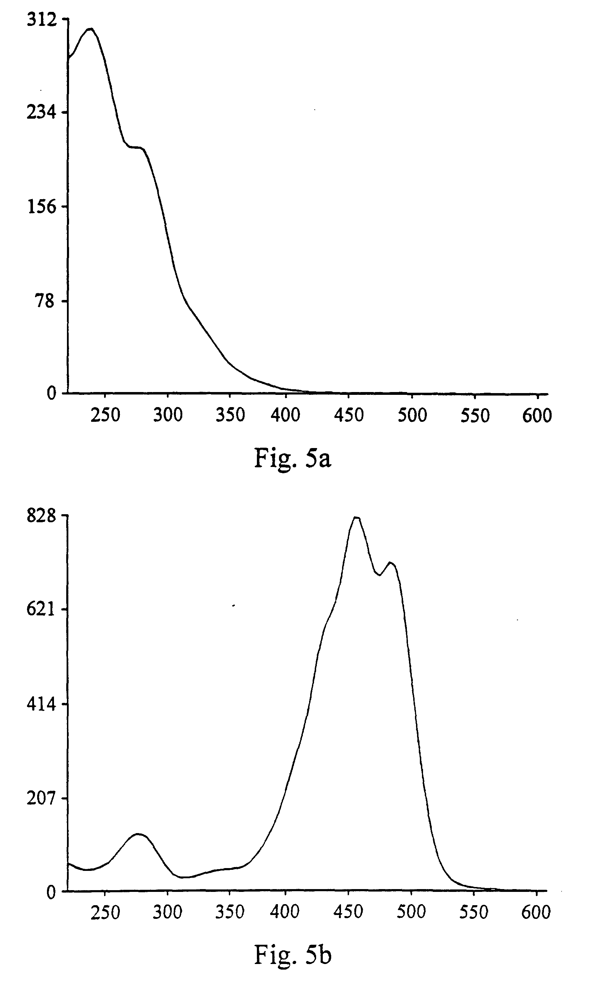

It was estimated from the weights of the individual fractions and GPC traces that the polymeric material accounted for close to 90 wt.% of the mixture of products of β-car^{ox}. The percentage of oxygen in the first three fractions obtained (i.e., the majority of the product) reflects well the 6-8 molar equivalents of oxygen taken up by the β-carotene and the 30-35% net increase in weight accompanying formation of the products. Addition of 6-8 molecules of oxygen to β-carotene, originally comprised of 11 conjugated double bonds, implies loss of most of the system of conjugated double bonds as most of the double bonds will have been disrupted by the formation of new carbon-oxygen bonds. Thus, the ultra violet-visible absorption spectrum of the polymeric substances show maximum absorption at a much shorter wavelength (ca 240 nm with a shoulder at 280 nm) compared to the parent β-carotene (Fig. 5a and Fig. 5b correspondingly).

The polymeric material, which is soluble in THF, methanol, acetone and acetonitrile, is stable indefinitely below room temperature but partially decomposes under heating forming volatile products as found by way of gas chromatography.

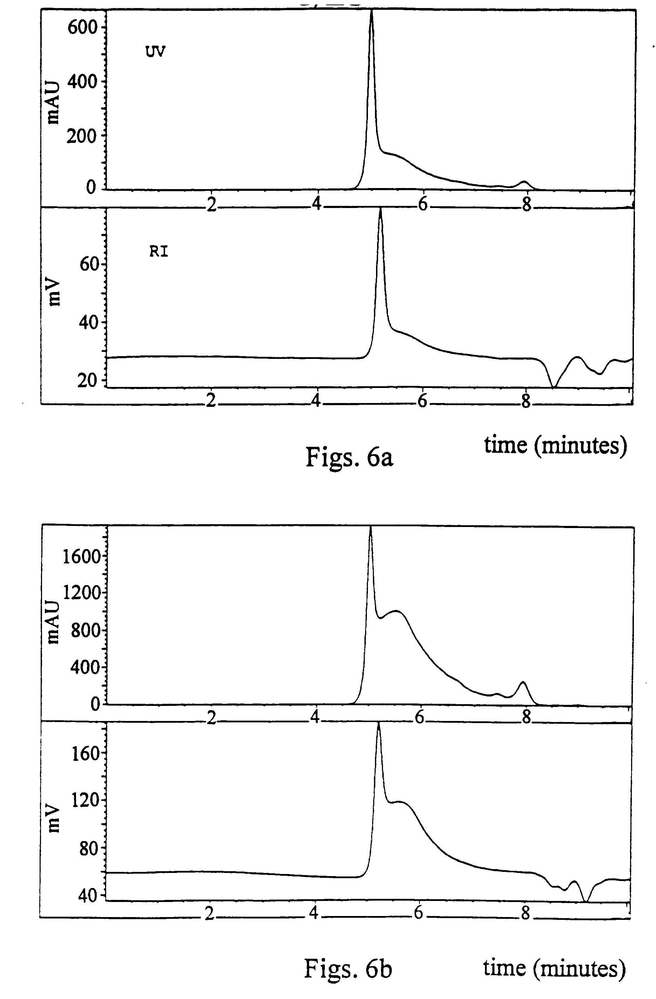

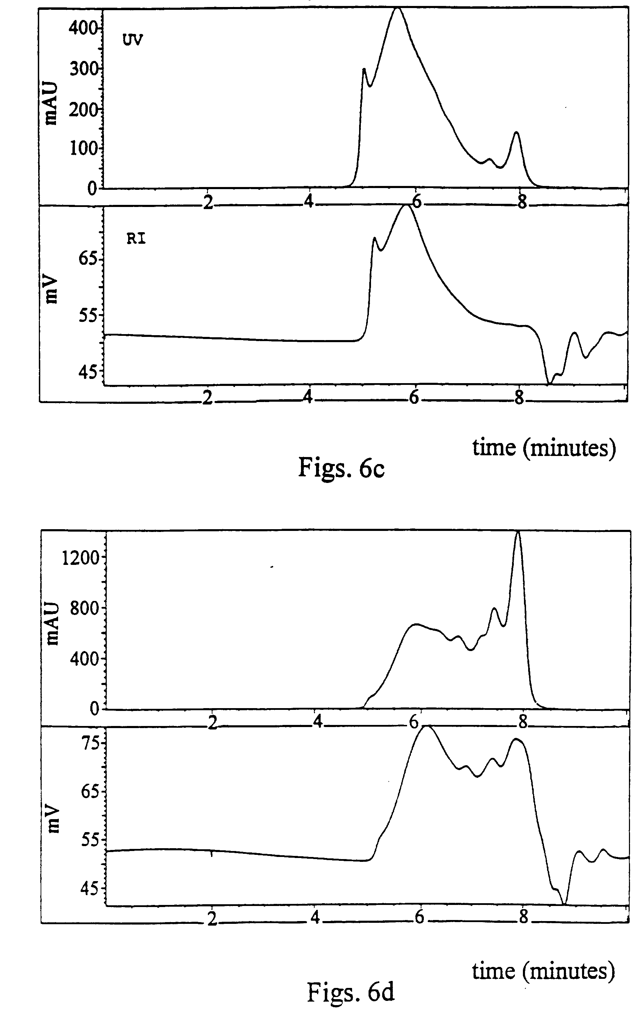

The GPC analysis of the specific fractions, 1-4, of the oxidation mixture (Figs 6a - 6d respectively), in comparison with Fig. 2, demonstrates that fractions 1 and 2 contain relatively large amounts of higher MW compounds while fractions 3 and 4, and particularly fraction 4, contain substantial amounts of lower MW materials.

### Oxidation of β-Carotene in Presence of Antioxidants

The oxidation of β-carotene (20 mM) also was carried out under the conditions described above but in the presence of 0.01 to 0.10 molar equivalents (with respect to β-carotene) of either alpha-tocopherol or 2,6-di-t-butyl-4-methoxyphenol. The reaction was slowed down considerably (1 molar equivalent of oxygen was consumed in ca. 6 days). The oxygen uptake plot was linear. The slope of this line was independent of the inhibitor type and its concentration. The β-carotene consumption did not exceed 10 % over a 6 day period.

### Oxidation of β-Carotene in Presence of Free Radical Initiator

2,2'-Azo-bis(2-methylpropionitrile) accelerated formation of reaction products.

### Solid State Oxidation of β-carotene:

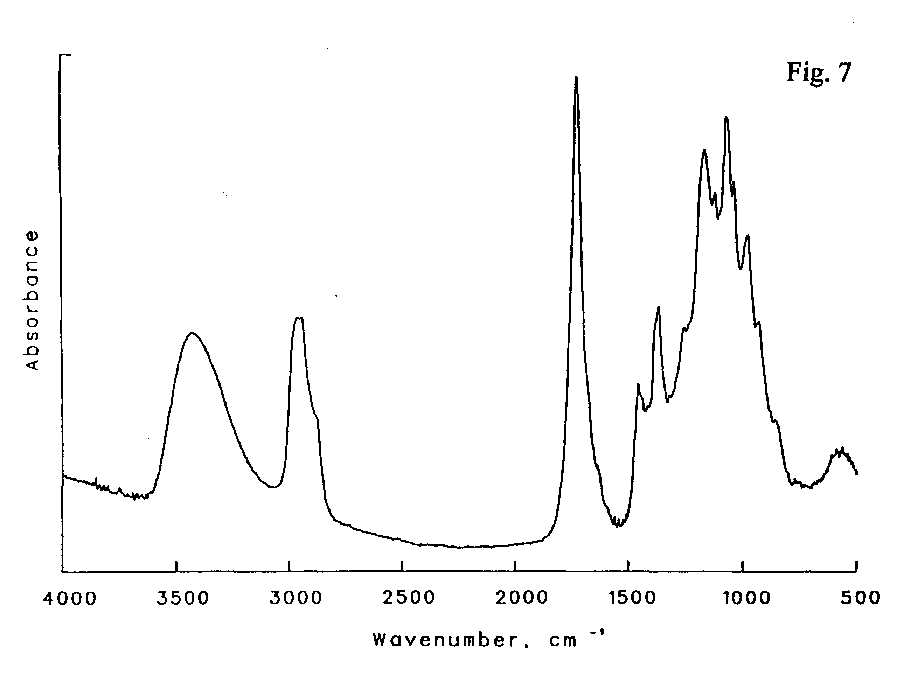

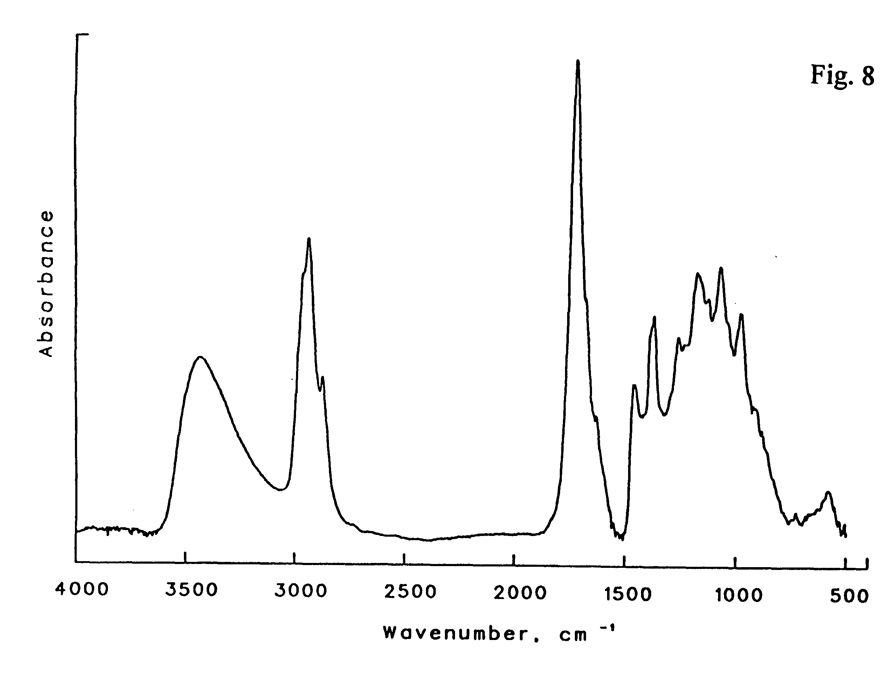

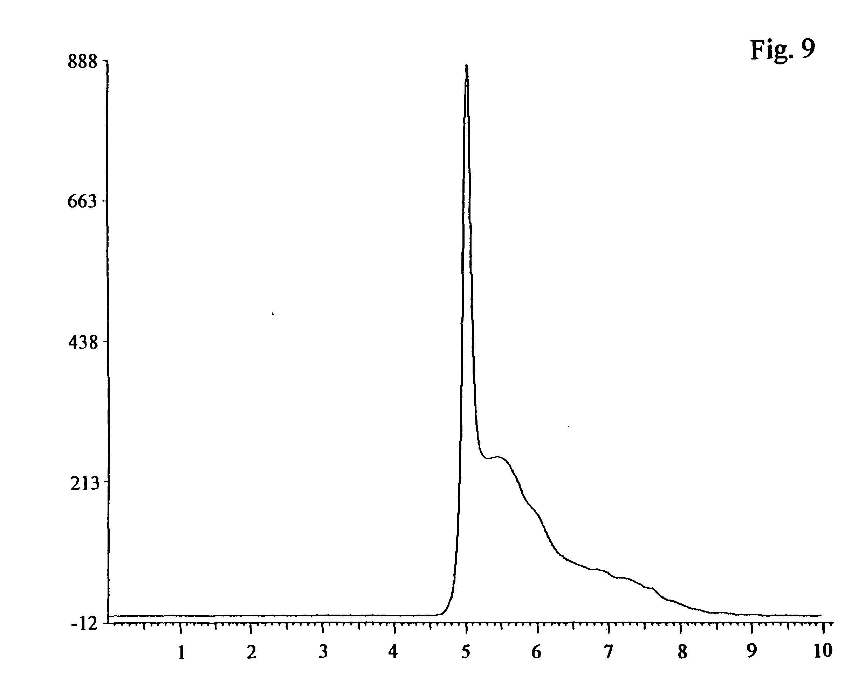

A similar polymeric material was obtained as virtually the only product, as indicated by FTIR and GPC analysis, when pure, crystalline carotene was oxidized in the solid state. The reaction was carried out by allowing crystalline β-carotene to stand in air over a period of up to 8 weeks in an open, clear glass vessel with no attempt to exclude light during daylight hours. The reaction was considerably slower than the reaction carried out in solution (40 days vs. 3 days). The FTIR spectrum of the polymer obtained by oxidation of solid β-carotene (Fig. 8) and the FTIR spectrum of fraction 1 of the polymer obtained by oxidation in solution (Fig. 7) were clearly similar regarding the location and relative intensities of the absorption peaks. The same was true for the results of GPC analysis (Fig. 9 vs. Fig. 6a). Furthermore, the substances obtained by oxidation of β-carotene in the solid state, and from partial and extensive oxidation in solution displayed similar biological activities in inhibiting the proliferation of cancerous and transformed cells grown in culture.

### Example 2: Oxidation of canthaxanthin

Canthaxanthin was oxidized under conditions identical to those used for the oxidation of B-carotene. Thus, a 20 mM solution of canthaxanthin (Fluka) in benzene saturated with oxygen was incubated in a shaker bath, in the dark, at 30°C under pure oxygen at atmospheric pressure. After 190 hours, when ca. 7 molar equivalents of oxygen was consumed, the solvent was evaporated to give a resin-like, yellow residue. Thus, although the reaction was slower, again there was very extensive reaction with oxygen. GPC analysis of the oxidation mixture (data not shown) indicated strong similarities with extensively oxidized β-carotene, i.e., the reaction products were predominantly polymeric substances.

### Example 3: Oxidation of retinoic acid

Reaction of retinoic acid with oxygen under the conditions used for β-carotene and canthaxanthin proceeded very slowly. The reaction was accelerated by carrying it out at elevated pressure.

Retinoic acid (Sigma) dissolved in benzene (0.5 ml, 20 mM) in a glass test tube was placed in a high pressure apparatus constructed of INCONEL 600. The apparatus was pressurized to 300 psi oxygen and placed for 3 days in a temperature-controlled bath set at 42 C. HPLC analysis of the reaction mixture showed that the reaction was incomplete. Reaction was continued for a further 2 days under the same conditions, except that the temperature was increased to 50 C, for a total reaction time of 5 days. Very little unoxidized retinoic acid remained (less than 1% of the total product). GPC analysis (data not shown) indicated the presence of higher MW products which, however, represented a much smaller fraction of the total reaction product than was found for β-carotene and canthaxanthin, and the absence of detectable material with MW greater than the cut-off of the column (approximately 1400 Dalton).

### Biology

In vitro biological assays were carried out by testing for cytostatic activity, induction of differentiation, and activity against tumorigenic viruses in various cultured cell lines. Most of the results were obtained for β-car^{ox} but also include some results for extensively oxidized canthaxanthin and retinoic acid.

In vivo tests were carried out by testing β-car^{ox} and extensively oxidized canthaxanthin for:
- toxicity in mice;
- inhibition of growth of tumors in mice including histopathological examination of tumor changes (tumor derived from a transplanted, chemically-induced rat mammary cancer cell line).

### In vitro tests

In order to determine the biological properties of the mixture of the invention, β-car^{ox} was tested in vitro for cytostatic effects and induction of differentiation. Retinoic acid and/or β-carotene were used as controls in some of the cell lines tested in order to differentiate their effects from that of oxidized β-carotene. The influence of oxidized β-carotene on the cell cycle was also studied. All concentrations relating to activity of β-car^{ox} are expressed in micro molar equivalents of β-carotene.

### Cell Models: Cell lines and characteristics

A variety of cell lines were used to test the effect of β-car^{ox} on proliferation and/or differentiation. The lines are either established transformed cell lines or are isolated from tumors of patients suffering from cancer. In addition, two murine cell lines were used in which the cellular differentiation program is well defined with appropriate protein markers. For these two lines, matching clones transfected with the human papilloma virus type 16 (HPV16) - a virus associated with cervical cancer - have been characterized and shown to exhibit transformation. The transformed phenotypes L6-HPV16 (derived from L6 cells) and BALB/c/MK-HPV16 (derived from BALB/c/MK cells) have been characterized by their hormone independence and ability to form colonies on soft agar.

The pattern of expression of the virus proteins in biopsies obtained from patients with severe neoplasia was established in our laboratory using the reverse transcriptase polymerase chain reaction (RT-PCR). In the biopsied material, the pattern of expression is similar to that of the transformed L6 clone, demonstrating the relevance and validity of the L6 model for testing purposes.

The BALB/c/MK cell line is of interest since it differentiates upon exposure to high concentrations of Ca²⁺ ions. When cells are exposed to low calcium, they revert to their pre-differentiation state in less than 72 hr.

The differentiation inducing properties of β-car^{ox} were further investigated in two additional models which used mouse embryonic stem cells (i.e., a quasi normal cell line) and several animal and human neuroblastoma cell lines (i.e., cancerous cells).

Embryonic stem (ES) cells are totipotent, i.e., they can give rise to any cell lineage of the organism. In vitro, under certain conditions, they differentiate spontaneously into a variety of mixed types. Treatment with substances known to promote differentiation directs ES cells towards a single phenotype. For example, retinoic acid prompts ES cells cultured under conditions established at the National Research Council to differentiate into neurons (unpublished data).

### Murine Cell Models

1a. L6 rat primary myoblasts (L6).
1b. L6 cells transfected with human papilloma virus type 16 (L6-HPV16).
2a. Mouse BALB/c/MK keratinocytes (BALB/c/MK).
2b. Mouse BALB/c/MK transfected with human papilloma virus type 16 (BALB/c/MK-HPV16).
3a. Mat B-WT: rat mammary adenocarcinoma.
3b. Mat B-MLNr rat mammary adenocarcinoma resistant to melphalan.
3c. Mat B-DOXr rat mammary adenocarcinoma resistant to adriamycin.
4. B16 mouse melanoma.
5. DA-3 mouse mammary carcinoma induced by DMBA.
6. FDCP-1 Mouse myeloid leukemia.

The two cell lines, Mat B MLNr and Mat B-DOXr exhibit multidrug resistance; Mat B-WT is the wild type. They are poorly differentiated cells which do not express estrogen or progesterone receptors.

### Human Cell Models

7a. MCF7-WT human breast carcinoma.
7b. MCF7-ADRr human breast carcinoma resistant to adriamycin.

The cells are moderately differentiated and positive for estrogen and progesterone receptors.
8a. Uro 9 human urothelial carcinoma; well differentiated.
8b. Uro 10 human urothelial carcinoma; poorly differentiated.
9. L14 human lung adenocarcinoma; moderately differentiated (isolated in-house at the Lady Davis Institute of the Montreal Jewish Hospital from a patient tumor).
10. NB4 Human acute promyelocytic leukemia.

### Cytostatic Effect

Table 2 summarizes the results obtained with the MTT viability/toxicity assay from Promega (a metabolic test based on mitochondrial dehydrogenase activity). Viability was assessed at increasing concentrations (multiples of 2.5) of β-car^{ox}. The doses which halve the growth of the cell population, as compared to untreated controls, are reported as IC₅₀ values.

**TABLE 2.**

| **Cytostatic Effect of Extensively β-Car**^{**ox**} | | |
|---|---|---|
| Cell Lines | Origin | IC₅₀ [*µ*M] |
| MatB-WT | Rat mammary adenocarcinoma | 25.5 |
| Mat-MLNr | | 21.0 |
| B16 | Mouse melanoma | 12.4 |
| DA-3 | Mouse mammary carcinoma | 15.6 |
| FDCP-1 | Mouse myeloid leukemia | 7.5 |
| L6 | Rat embryonic myoblasts | 51.9 |
| L6-HPV16 | | 28.2 |
| MCF7-WT | Human breast carcinoma | 11.3 |
| MCF7-ADRr | | 11.3 |
| Uro-9 | Human urothelial carcinoma | 17.7 |
| Uro-10 | | 19.8 |
| L14 | Human lung adenocarcinoma | 18.5 |
| NB4 | Human promyelocytic leukemia | 4.8 |
| Exponentially growing cells cultured at low density (10⁴ to 10⁵ cells/1-5 ml) were exposed to a single dose of different concentrations of β-car^{ox} for 72 hr or more. Cell growth was determined by the MTT test. IC₅₀ values (expressed in β-carotene equivalents) were determined graphically from survival curves (plots of cell growth versus concentration of β-car^{ox}). Each value in the Table 2 corresponds to an average of at least two independent experiments. Different batches of extensively oxidized β-carotene were tested and gave consistent results. Note the sensitivity of the NB4 leukemia cell line and the lack of activity on the L6 control cells. Retinoic acid and β-carotene (each 3 *µ*M), used as controls for some cell lines, were found to have no effect on the cell growth curves, except for NB4 cells which responded to retinoic acid. | | |

As a cross-check on the use of the MTT test, IC₅₀ results for L6 and L6-HPV16 cell lines were confirmed by cell enumeration using a Coulter counter as illustrated in Table 3.

**TABLE 3.**

| **Comparison of the cytostatic effect (IC**_{**50**} **[*µ*M]) of β-car**^{**ox**} **as measured by MTT test and cell enumeration by Coulter counter** | | |
|---|---|---|
| Cell Line | MTT | Coulter counter |
| L6 | 51.9 | 39.0 |
| L6-HPV16 | 28.2 | 24.6 |

The fractions obtained from β-car^{ox} after successive organic solvent precipitations were assayed on the MCF7-WT cell line; two fractions, 1 and 3 were found to be considerably more active than the crude mixture, as illustrated in Table 4.

**TABLE 4.**

| **Cytostatic effect of fractions against MC7-WT cell line (*µ*M)** | | | | | |
|---|---|---|---|---|---|
| Fractions | 1 | 2 | 3 | 4 | β-car^{ox} |
| MCF7-WT IC₅₀ | 11.8 | 17.3 | 13.1 | 26.2 | 22.8 |

Cells were treated as described in the footnote to Table 2.

Although normal cells appear to be mildly inhibited, over a longer period of time in culture this effect gradually disappeared, showing that β-car^{ox} does not drastically affect normal cells. This would potentially allow cancer patients to be treated repeatedly with limited side effects.

### Effect on Cell Differentiation

### Morphological Observations on the L6 myoblast models:

The cultured rat myoblasts, L6, are capable of differentiation in vitro. At the National Research Council, five steps have been characterized in the differentiation program of these cells using morphometry, in situ immunofluorescence and flow cytometry analysis.

The known markers (characteristic proteins) of myocyte differentiation used were: fibronectin, a-actin, N-CAM, vimentin and expression of acetylcholine receptors. In addition, it was demonstrated that acetylcholine receptors specific to the prefusion stage were not expressed in the L6-HPV16 cells. Five stages were identified, the phenotypes of which in order of increasing differentiation are described below:
1. Cells have embryonic fibroblast-like appearance.
2. Cells acquire a bipolar morphology.
3. Cells become oriented.
4. Cells enter pre-fusion stage.
5. Cells show formation of syncitium and myotubes.

It has been found that the HPV16 transfected cells are transformed and blocked at the pre-fusion stage. That is, HPV16 transfected cells are blocked at stage 4. Upon treatment with β-car^{ox}, however, better orientation of the cells was observed and some syncitium formation was initiated, corresponding to partial entry into stage 5 of differentiation.

Culturing in media differing in their calcium content showed that β-car^{ox} was more potent on both controls and transfected cells when the media were poor in calcium (0.05 mM). In addition, an antagonism was observed between calcium and β-car^{ox} in the expression of differentiation markers reported above. This prompted some exploratory experiments on the effects of calcium upon the cells treated with β-car^{ox}. The myoblast and keratinocyte models were used.

Briefly, in L6 myocytes, the influx of exogenous calcium in the cells occurs via nonspecific cationic channels (as we have established earlier and in agreement with existing literature). Inside the cells, the level of calcium is controlled by calcium release from internal stores. In addition, an acetylcholine receptor expressed at the pre-fusion stage of the L6 differentiation program modulates sarcoplasmic calcium channels.

In keratinocytes, the nature of the channels regulating calcium entrance and release from stores is not yet elucidated.

Experiments were conducted by two techniques: electrophysiology and imaging using Fura-2. Preliminary results indicate that β-car^{ox} acts as a calcium channel blocker or calcium chelator. This was confirmed by its ability to partly overcome multidrug resistance in the multidrug resistant cell lines described. This, however, appears less effective than conventional channel blockers like verapamyl.

### Pattern of the Differentiation Markers in the BALB/c/MK models

In BALB/c/MK keratinocytes, cytokeratins 1, 5 and 10, as defined in the Poll catalogue, were first identified by Western immunoblot. Cytokeratins 1 and 10 are known to be associated with higher levels of differentiation while cytokeratin 5 is associated with less differentiated, still proliferating cells.

Flow cytometry analysis showed that cytokeratins were generally less expressed in untreated HPV16 transfected cells than in untreated matching control cells.

### Cytokeratin 5

Exposure to 1.8 mM calcium, which induces irreversible differentiation within 3 days, resulted in an increase in the expression of cytokeratin 5 in the controls and the transformed cells. Exposure to β-car^{ox} resulted in a similar effect except that 6 days of exposure were sufficient to induce expression in the controls, while 9 days were necessary for the transformed cells.

Exposure to both inducers simultaneously, canceled the enhancement of expression, showing that the two compounds are antagonistic.

### Cytokeratins 1 and 10

A striking increase in expression of both of these cytokeratins upon exposure to increased calcium (from 0.05 mM to 1.8 mM) was observed in control cells while the transformed cells did not show any response.

In contrast, β-car^{ox} induced expression of both markers in *both* lines. As with the L6 line, longer exposure was required for a response from BALB-HPV16 as compared to controls.

Again, antagonism with calcium treatment was observed. These results seem of real importance because they provide evidence that, at least in the keratinocyte model, β-car^{ox} enhances differentiation in the HPV16 transformed cells whereas the classical calcium cell differentiation inducer is ineffective.

Induction of differentiation by β-car^{ox} (estimated by morphological criteria) also has been observed in the NB4 promyelocytic leukemia cell.

### ES and neuroblastoma cells

Under conditions similar to those developed at the National Research Council for the induction of differentiation by retinoic acid of ES cells into neurons, β-car^{ox} also promotes the differentiation of ES cells into neural cells as shown in Fig. 10 and as assessed by specific markers, using immunohistochemical techniques. However, there are two significant differences between the effect of retinoic acid and β-car^{ox} on ES cells.
1) Retinoic acid promotes ES cell differentiation into 80% of a bipolar phenotype in a dose-independent fashion in the 0.1 to 1 *µ*M range (Fig. 10b) while β-car^{ox}, at the optimal concentration (7.5 *µ*M β-carotene equivalents), elicits terminal differentiation into ca. 90% of highly branched phenotypes resembling Purkinje cells (Fig. 10d); the mechanism of high branching is not yet fully elucidated.
2) The induction of terminal differentiation is kinetically different for the two inducers; β-car^{ox} is far more effective than retinoic acid (15 hr versus 3 days, respectively, under our experimental conditions)

In the neuroblastoma cell lines Neuro2A, IMR32, SK-N-SH and SK-N-MC β-car^{ox} promoted similarly striking high degrees of differentiation, whereas retinoic acid evoked only partial differentiation.

The results from the ES and neuroblastoma models indicate that β-car^{ox} is a powerful differentiation promoter and it appears that its mechanism of action is different from that of retinoic acid.

### Activity against expression of tumorigenic viral genes

The observation that β-car^{ox} may possess activity against the expression of tumorigenic viral genes relies on three independent experiments.

Cytopathy: The symptoms of viral infection, clearly visible as intense vacuolization around the nuclei of the transfected cells L6-HPV16 and BALB/c/MK-HPV16, drastically decreased or disappeared upon exposure to β-car^{ox}.

Messenger RNA was extracted from transfected cells that had and had not been exposed to the β-car^{ox}. The kinetics of the expression pattern of the viral gene products has been analyzed by RT-PCR. Initial results showed that the pattern of expression of the viral proteins and the myc oncogene (an oncogene related to proliferation) was different for cells treated with β-car^{ox}, compared to those treated with retinoic acid or β-carotene.

A monoclonal antibody raised against a fragment common to the E6 and E7 oncogenic proteins of HPV16 was used to determine the expression of both proteins using polyacrylamide gel electrophoresis, Western blot analysis and flow cytometry. A decrease of the expression of E6 and E7 proteins was observed after 9 to 12 days exposure of the virus transfected cells to β-car^{ox}.

### Cytostatic effect of incompletely oxidized β-carotene, antioxidant-inhibited β-carotene oxidation mixture, and extensively oxidized canthaxanthin and retinoic acid:

Table 5 shows the relative effects towards the MC7-WT cell line of incompletely oxidized β-carotene containing both the polymeric oxidation product and unreacted β-carotene, the oxidation products obtained from the oxidation of β-carotene inhibited by a-tocopherol (5 mole %), fully oxidized canthaxanthin and retinoic acid (syntheses described above). All of the samples contained at least some of the higher MW oxidation materials and all showed considerable cytostatic activity.

**TABLE 5.**

| **Cytostatic activity against MC7-WT cell line of partially oxidized β-carotene, products obtained in inhibited oxidation of β-carotene, as well as extensively oxidized canthaxanthin and retinoic acid** | |
|---|---|
| Sample | IC₅₀ (*µ*M) |
| ca. 25% Oxidized β-carotene | 22 |
| ca. 50% Oxidized β-carotene | 21 |
| 100% Oxidized β-carotene | 17 |
| a-Tocopherol-retarded oxidation of β-carotene | 11 |
| Oxidized canthaxanthin | 9 |
| Oxidized retinoic acid | 7 |

Cells were treated as described in the footnote to Table 2.

### In Vivo Study of Toxicity and Anti-Tumor Activity of β-Car^{ox}

### Assessment of Toxicity: β-Car^{ox} and extensively oxidized canthaxanthin

Toxicity was assessed by monitoring body weight of female BALB/c mice and by general examination of the animal. Dosages of 5 mg/kg and 10 mg/kg were injected intraperitoneally on days 1, 3 and 5 (Table 6). The control groups received solvent only (20% aqueous ethanol). A similar study was carried out using 50 and 100 mg/kg injections on days 1,3,5,8 and 11. No overt toxic effects were observed (data not shown).

β-Car^{ox} is non-toxic to healthy mice even when applied in six doses of 100 mg/kg. It should be stressed that even with repeated doses (9 times) of up to 150 mg/kg (with tumor bearing mice), no adverse effects have been observed.

Similarly, extensively oxidized canthaxanthin showed no overt signs of toxicity under an identical dose/injection pattern regime (data not shown).

**Table 6**

| . **Effect of extensively oxidized β-carotene on body weight of mice** | | | |
|---|---|---|---|
| Day | Average Body Weight [g] | | |
| | Control | 5 mg/kg | 10 mg/kg |
| 1 | 14.0 | 14.4 | 14.0 |
| 2 | 14.0 | 14.5 | 14.0 |
| 5 | 14.4 | 14.9 | 14.5 |
| 7 | 14.8 | 15.3 | 14.6 |
| 9 | 15.2 | 15.8 | 15.2 |
| 11 | 15.7 | 16.3 | 15.7 |
| 13 | 16.1 | 16.7 | 16.2 |
| 15 | 16.6 | 17.3 | 16.9 |
| 17 | 17.1 | 17.7 | 17.4 |
| 19 | 17.6 | 18.3 | 17.9 |
| 21 | 18.1 | 18.7 | 18.4 |
| 23 | 18.7 | 19.4 | 19.1 |
| 25 | 19.2 | 20.0 | 19.8 |

### Anti-Tumor Activity: Tumor Model System

The mouse D1-DMBA-3 (DA-3) mammary adenocarcinoma model was used. The cell line was derived from BALB/c mice bearing an immunogenic non-metastatic, murine mammary adenocarcinoma induced by 7,12-dimethylbenzanthracene (DMBA).

One million DA-3 cells were injected subcutaneously into each female BALB/c mouse. When the tumors became palpable (0.5 cm diameter, 1-2 weeks) the animals were randomized into groups and injected intraperitoneally with the β-car^{ox} at dosages ranging from 5 mg/kg up to 150 mg/kg. Tumor growth was measured every 2-3 days. The evaluation was carried out by determining inhibition of tumor growth by measuring tumor volume as a function of time, as described by Alaoui-Jamali et al., in J. Pharmacol. Exp. Ther. **1993**, 264 (3), 1299. Again, the control group received solvent only (20% aqueous ethanol).

Fig. 11 and Fig. 12 illustrate the effect of β-car^{ox} on tumor growth. Fig. 11 corresponds to a dose of 10 mg/kg injected on days 0,2,4,7,9,11,14,16 and 18. Fig. 12 corresponds to a dose of 150 mg/kg injected in the same way.

For histological examination, a few, randomly chosen animals were sacrificed and tumors were dissected and fixed in 10% formalin in normal saline. Histological sections were prepared from each of the formalin-fixed, paraffin-embedded tumors and stained with hematoxylin-eosin.

The results show that β-car^{ox} has a growth-retarding effect on a cancer cell-derived tumor implanted in mice. Fig. 11 and 12 show that extensively β-car^{ox} as applied repeatedly at dose as low as 10 mg/kg has the ability to effectively arrest the growth of the tumor and stabilize it for a long period of time.

Fig. 13a and Fig. 13b show the comparison of tumors in sacrificed animals which had been treated with different doses of β-car^{ox} and the control (untreated animal). In some animals hemorrhaging occurred around the tumors (Fig. 13b). In these cases the actual tumor size is smaller than when measured with calipers (the discrepancy is attributed to hemorrhagic swelling increasing the apparent tumor size).

Histopathological examination of tumors removed from treated animals revealed that:
- β-car^{ox} induces pronounced histological alterations, reflecting tissue death in DA-3 tumors.
- tumor tissues showed many features of cell/tissue differentiation.
- hemorrhagic areas are present in all treated tumors and are associated with extensive pigmentation and necrosis.
- the pigments are not iron (iron staining using Prussian blue was negative), but may be hemosiderin probably resulting from hemorrhagia. It appears unlikely that the pigment is melanin. The exact nature of the pigmentation remains to be confirmed.

There was no evidence of similar histopathological changes in normal, non-tumor tissues.

As multiple intraperitoneal injections of the β-car^{ox} are well tolerated, even at concentrations of up to 150 mg/kg, the therapeutic index of β-car^{ox} appears to be very high, which potentially offers a major advantage over traditional anti-cancer drugs.

To summarize the first phase of our research, β-Carotene and canthaxanthin, as representative carotenoids, and to a lesser extent, retinoic acid, a representative retinoid, can undergo extensive oxidation to yield substances, insofar as β-car^{ox} is a model, which demonstrate properties that make the substances useful as non-toxic agents active against uncontrolled cell proliferation, tumors, and tumorigenic viruses, and useful as promoters of cell differentiation. It is evident from chemical analysis of β-car^{ox} that none of the various forms of vitamin A are present or are present only in minor amounts. Furthermore, the biological activities of oxidized canthaxanthin and retinoic acid, which cannot form vitamin A, indicate the presence of active substances that are different from vitamin A. Although the cytostatic and differentiation-promotion activities of β-car^{ox} resemble those of vitamin A itself, generally the effects are more powerful for β-car^{ox} in a wide variety of circumstances. Also, there is the very important difference that β-car^{ox} is non-toxic.

### SECOND PHASE OF RESEARCH

### Chemistry

### Oxidation of lycopene

Lycopene (see Fig. 14) dissolved in benzene was extensively oxidized under an atmosphere of oxygen in essentially the same manner as β-carotene in the first phase of work. The crude product mixture was tested for cytostatic activity in a manner analogous to β-car^{ox}.

### Fractionation of β-Car^{ox}

The fractionation evolved through three phases. The three separation schemes, 3-4 levels deep, are depicted below:

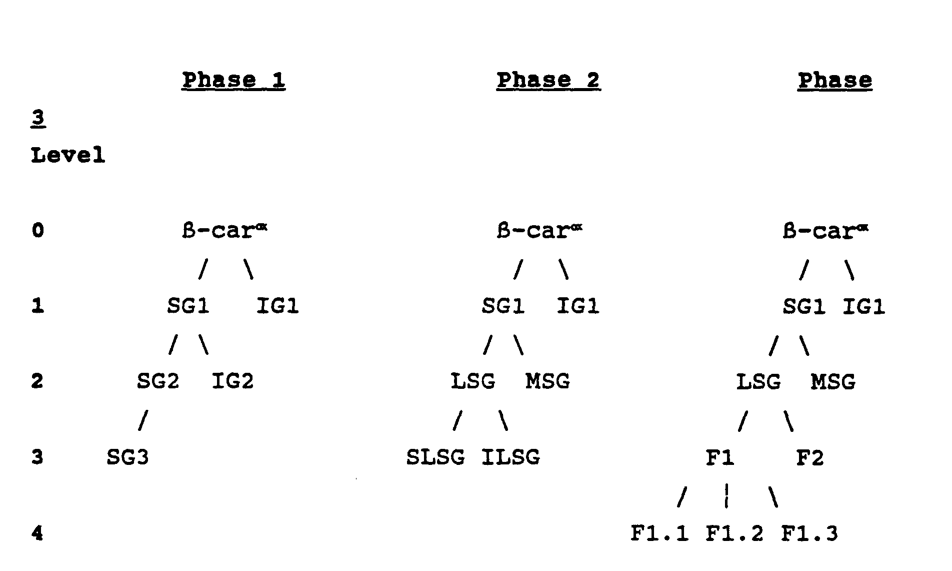

β-Car^{ox} was synthesized using a procedure very similar to that described in the first phase. Briefly, β-carotene (30 g) dissolved in benzene (3.0 1; 0.02 M) was stirred for 4 days at room temperature under an atmosphere of gaseous oxygen. GPC chromatography confirmed the presence of three major components, i.e., the low (< 300 Da), medium (300-1000 Da), and high (> 1000 Da) MW fractions (Figure 15).

Level 1 separation (phases 1-3): the solution (3.0 l) was concentrated to ca. 200 ml and then diluted with approximately 2 l of hexane. The precipitate, **IG1,** ca. 65% of the total mixture, contained most of the high MW material (> 1000 Da). The supernatant containing the soluble fraction, i.e., the low (< 300 Da) and medium (300-1000 Da) MW fractions and practically none of the high MW fraction (see Figure 16), was evaporated to dryness to give a residue, **SG1**.

Level 2 separation (phase 1): fraction SG1 (1.2 g) was stirred in hexane (120 ml) at room temperature for 30 min. The insoluble fraction, **IG2**, that was filtered off, contained mostly medium MW and some low MW material (Figure 15). The supernatant containing the soluble fraction was evaporated to yield **SG2,** containing mostly low MW and some medium MW material (Figure 16).

Level 2 separation (phases 2 and 3): fraction SG1 (10 g) dissolved in tetrahydrofuran (10 ml) was separated into low and medium MW fractions, **LSG** (40%) and **MSG** (55%), respectively, by successive injections of samples (250 *µ*l) onto a preparative-scale GPC chromatography column (19 x 300 mm, Waters styragel, 15 *µ* m particle size, 10 nm pore size) and elution with tetrahydrofuran (4 ml/min). A clean separation into two MW fractions was obtained as illustrated in Fig. 17.

Level 3 separation (phase 1): fraction SG2 (688 mg) was loaded onto a silica gel column and eluted with hexane/ethyl acetate (95:5). The eluted fractions were combined and the solvent removed to give fraction **SG3.** Level 3 separation (phase 2): fraction LSG (600 mg) was stirred with ice-cold pentane (1 ml) for 1 min. and then most of the supernatant was decanted off. This procedure was repeated four times. The insoluble fraction, **ILSG,** was filtered and the solvent was evaporated from the combined supernatant fractions containing the soluble component, giving fraction **SLSG.**

Level 3 separation (phase 3): fraction LSG (4.4 g), dissolved in acetonitrile (10 ml), was separated into two fractions by successive injections (450 *µ*l; total 9.0 ml) onto a *preparative*-scale HPLC instrument equipped with three Waters NovaPak HR C18 (reverse phase, 6 *µ*m particle size, 6 nm pore size) PrepPak cartridges (40 x 100 mm) connected in series and eluted with acetonitrile (40 ml/min). Fractions **F1** (80%) and **F2** (20%) were obtained by collecting eluent from 4.8 to 6.4 min and 6.4 to 12 min, respectively. The dotted vertical line in the middle of the *analytical* high performance liquid chromatography (HPLC) chromatogram in Figure 18 shows how LSG was divided into the two new fractions, F1 and F2. (The elution times of compounds in Figure 18 and the corresponding division time are different from those in the *preparative* HPLC because of differences in the conditions necessary to obtain optimal separations under *analytical* and *preparative* conditions, respectively.)

Level 4 separation (phase 3): fraction F1 (700 mg), dissolved in acetonitrile (1.5 ml), was separated into three fractions, **F1.1** (11%), **F1.2** (13%) and **F1.3** (5%), by successive injections (250 *µ*l; total 1.25 ml) onto a preparative-scale HPLC instrument equipped with the same column already described and eluted with a 50:45:5 mixture of water, acetonitrile and methanol at a flow rate of 40 ml/min. The fractions were obtained by collecting eluent from 2.0 to 6.6 min, 6.6 to 12.0 min and 12.0 to 23 min, respectively. The dotted vertical lines in Figure 19 show how the cuts were made to obtain the three fractions.

### Determination of the Chemical Composition of Some Fractions at the 3rd and 4th Levels of Separation

### (Structures of numbered compounds are provided in Figure 21.)

Fractions F1.1, F1.2 and F1.3 each contain only a few compounds as Figure 19 indicates. A major component in **F1.2** is 2-methyl-6-oxo-2,4-heptadienal (compound **1**, referred to elsewhere in the text as ketoaldehyde). Dihydroactinidiolide (compound **2)** is a major component of **F1.3.** Compounds **3-14** have been identified in fraction **F2.** These are β-cyclocitral **(3),** β-ionone **(4),** β-ionone 5,6-epoxide **(5),** 4-oxo-β-ionone **(6),** β-ionylidene acetaldehyde **(7),** β-ionylidene acetaldehyde 5,6-epoxide **(8),** 4-oxo-β-ionylidene acetaldehyde **(9),** β-apo-13-carotenone (**10**), β-apo-13-carotenone 5,6-epoxide (**11**), 4-oxo-β-apo-13-carotenone (**12**), retinal (**13**) and retinal 5,6-epoxide (**14**).

### Biology

In vitro biological assays were carried out by testing for cytostatic activity, induction of differentiation, and the effect upon the cell cycle. In vivo tests were carried out by testing for inhibition of growth of tumors in mice.

### In Vitro Tests

Fractions were tested for cytostatic properties and ability to induce differentiation. The results were compared with those obtained for β-car^{ox}, which served as a reference indicating whether or not fractionation was leading to fractions with enhanced activity. (Retinoic acid and/or β-carotene were used as controls in some of the cell lines tested in order to differentiate their effects from that of β-car^{ox}). Given the present indeterminate nature of many of the fractions, all samples were tested in identical 'by weight' concentrations. Comparisons have been done on a pseudo molarity scale by dividing the weight of sample used by the molecular weight of β-carotene (537 Da).

Cell lines used:

| | |
|---|---|
| HCT116 | human colon carcinoma |
| IMR32 | human neuroblastoma |
| NB4 | human acute promyelocytic leukemia |
| K562 | human chronic myelogenous leukemia |
| BALB/c/MK | mouse keratinocytes |

### Cytostatic Effect of β-Car^{ox} Fractions

Cells were treated with six concentrations of each sample (2.5, 7.5, 15, 22.5, 30, 45 *µ*M for HCT116 and IMR32 and 2.5, 5, 7.5, 10, 15, 22.5 *µ*M for NB4 and K562 cell lines). After 3 days, the cells were lysed, incubated with Hoechst dye 33258 and the fluorescence of the solution was measured to provide a measure of the amount of cellular DNA present. The fluorescence data for each substance tested were collected and divided by the corresponding values for untreated cells (after correcting for background fluorescence) to determine the cytostatic effect for each sample concentration. Figures 22a and 22b illustrate results that were obtained for the HCT116 human colon cancer and the K562 human leukemia cell lines respectively.

It is apparent that relative activity can depend very much upon the cell line chosen. This is illustrated particularly well by fraction MSG which is active in the HCT116 cell line but is inactive in the K562 line, whereas fraction ILSG is highly active in both lines (more so than β-car^{ox}). These observations imply that the cytostatic effect can be obtained through the action of more than one compound.

Table 7 summarizes the results that were obtained for the four cell lines. Fractions have been ranked relative to β-car^{ox} by dividing each growth inhibition value at each sample concentration by the corresponding β-car^{ox} value and determining qualitatively by inspection the trends across the six sample concentrations. Activities of fractions are expressed as "+", "o", and "-", indicating activity higher, similar to, and smaller than, respectively, the activity of β-car^{ox}.

One of the most striking results is the strong inhibition of cell growth obtained for the ketoaldehyde (compound **1** in Figure 21), which has been identified as a major component of fraction F1.2. Perhaps surprising is the apparent diminution of activity in the simpler fractions at more advanced levels of fractionation (3 and 4) following an enhancement of activity in their more complex parent fractions at preceding levels (1 and 2), e.g., fractions F1, F2, F1.2 and F1.3. The later, simpler fractions should, in principle, be enriched in active components, leading to the expectation of higher activity. It is possible that the higher activity observed in the earlier, more complex fractions derives from the presence of more than one active compound and/or from synergistic relations between two or more compounds.

**Table 7.**

| Cytostatic effect upon several cell lines of fractions and selected compounds expressed relative to β-car^{ox}. | | | | | |
|---|---|---|---|---|---|
| Stage | Fraction | Cell Line | | | |
| | | HCT116 | IMR32 | K562 | NB4 |
| 0 | β-Car^{ox} | o | o | o | o |
| | | | | | |
| 1 | SG1 | o | + | + | + |
| | IG1 | + | o | o | + |
| | | | | | |
| 2 | SG2 | + | + | + | + |
| | IG2 | + | + | + | + |
| | | | | | |
| 2 | LSG | - | o | + | o |
| | MSG | o | o | - | o |
| | | | | | |
| 3 | SG3 | + | + | + | o |
| | | | | | |
| 3 | F1 | - | o | o | o |
| | F2 | o | o | o | - |
| | | | | | |
| 4 | F1.1 | - | o | + | + |
| | F1.2 | - | o | - | o |
| | F1.3 | - | o | o | o |
| | | | | | |
| | Ketoaldehyde | + | + | + | + |
| | | | | | |
| | β-Car | - | o | - | - |
| | RA | - | + | o | o |

NOTE:
Data shown above were calculated using a value for β-Car^{ox} that was averaged over all assays. The values for β-Car^{ox} are, by definition, set at "o". RA = *all-trans* retinoic acid.

### Cytostatic Effect of Extensively Oxidized Lycopene

The activity of extensively oxidized lycopene was compared to that of β-car^{ox} using the HCT-116 cell line. The results in Table 8 show the two substances have similar activity against this cell line. We have shown that β-car^{ox}, canthaxanthin and now lycopene yield products which have strong cytostatic properties. As lycopene lacks the two cyclohexyl rings present in the other two carotenoids (see Figure 14), it appears that the presence of this molecular component is not necessary for cytostatic activity in the oxidation products.

**Table 8.**

| Comparison of cytostatic effect of extensively oxidized lycopene and β-car^{ox} in the HCT-116 cell line. | | |
|---|---|---|
| Dose [*µ*M] | β-Car^{ox} | Oxidized Lycopene |
| 0 | 100 | 100 |
| 2.5 | 86 | 100 |
| 7.5 | 80 | 85 |
| 15 | 51 | 55 |
| 22.5 | 18 | 41 |
| 30 | 8 | 18 |
| 45 | 1 | 11 |

### Cell Differentiation

The cell differentiation-inducing effects of the fractions were determined qualitatively by observation of morphological changes in cell lines and quantitatively by measuring the expression of characteristic protein markers using monoclonal antibodies and flow cytometry. In Table 9, active fractions are denoted by the "+" symbol and highly active fractions are denoted by a "++".

There is widespread activity among the fractions in the IMR32 human neuroblastoma cell line. Unexpectedly, three pairs of fractions (SG1, IG1; SG2, IG2 and LSG, MSG) show an unprecedented ability (as far as we are aware) to induce differentiation towards two different phenotypes from the same precursor cell line. That is, it is possible to direct the IMR32 cell line towards glial or neuronal cells, depending on the choice of fraction. The ability to induce formation of neurons disappears in the later, simpler fractions (Stage 4; Table 9). These observations are further evidence that β-car^{ox} contains multiple active compounds possessing different types of activity.

Differentiation induction in the NB4 human leukemia cell line is confined to the early and chemically complex IG2 and MSG fractions which are strongly active. Only one fraction, SG3, potentiates differentiation in the quasi-normal L6 rat myoblast cell line.

The greater activity in the transformed cell lines, particularly IMR32, compared to the quasi-normal L6 line provides support for the strategy of using selective differentiation of transformed cells to control cancerous cell growth in a non-toxic manner.

**Table 9.**

| Differentiation inducing effects of β-car^{ox} fractions. | | | | |
|---|---|---|---|---|
| Stage | Fraction | Cell Line^{a} | | |
| | | IMR32^{b} | NB4 | L6 |
| 1 | SG1 | +(n) | ne | ne |
| | IG1 | +(g) | ne | ne |
| | | | | |
| 2 | SG2 | +(n) | ne | ne |
| | IG2 | +(g) | ++ | ne |
| | | | | |
| 2 | LSG | ++(n) | ne | ne |
| | MSG | ++(g) | ++ | ne |
| | | | | |
| 3 | SG3 | +(n) | ne | ++ |
| | | | | |
| 3 | F1 | ++(n) | ne | ne |
| | F2 | ++(n) | ne | ne |
| | | | | |
| 4 | F1.1 | ++(g) | ne | ne |
| | F1.2 | ne | ne | ne |
| | F1.3 | ne | ne | ne |
| | | | | |
| | Ketoaldehyde | ne | ne | ne |

| | | | | |
|---|---|---|---|---|
| ^{a} ne = no effect | | | | |
| ^{b} Letters in parentheses indicate differentiation towards glial (g) or neuronal (n) phenotypes. | | | | |

Qualitative data were provided in the first phase of research for the differentiation-inducing effect of β-car^{ox} upon the mouse Balb/c/MK keratinocyte cell line. Tables 10 and 11 now provide supporting quantitative data.

**Table 10.**

| Expression of cytokeratins 1 and 10 in Balb/c/MK keratinocytes. | | |
|---|---|---|
| Sample | A | I_{A} |
| Control | 90.3 | 4.4 |
| | | |
| Ca⁺⁺ (1.8 mM) | 96.5 | 13.8 |
| | | |
| RA (3 *µ*M) | 94.5 | 15.4 |
| β-Car^{ox} (30 *µ*M) | 96.5 | 7.9 |

Note: Column A is the percentage of immunopositive cells, i.e., expressing the indicated cytokeratins. Column I_{A} represents the intensity of expression per cell (arbitrary units). RA = all-trans retinoic acid.

**Table 11.**

| Expression of a panel of cytokeratins in Balb/c/MK keratinocytes. | | |
|---|---|---|
| Sample | A | I_{A} |
| Control | 93.7 | 60.7 |
| Ca++ (1.8 mM) | 97.6 | 87.2 |
| RA (3 *µ*M) | 94.3 | 129.8 |
| β-Car^{ox} (30 *µ*M) | 96.2 | 82.1 |

Note: see footnote to Table 10

Cytokeratins 1 and 10 are high molecular weight proteins which are increasingly expressed with the progression of the differentiation program. In in vitro cell cultures, the differentiation is classically induced by exposure to culture media containing high levels of calcium. Cells cultured in low calcium media remain proliferative. As illustrated in Table 10, retinoic acid can substitute for Ca⁺⁺ in increasing expression of cytokeratins 1 and 10. β-Car^{ox} also can substitute for calcium in inducing Balb/c/MK keratinocyte differentiation. The apparently smaller effect of β-car^{ox} compared to retinoic acid is due not to lower potency but to a delayed action. Table 11 shows that similar results have been obtained using a panel of other cytokeratins.

### Combined effect of β-car^{ox}, or its fractions, with all-trans retinoic acid towards a) identifying the cellular target of β-car^{ox} and b) combination therapy.

Given the cell differentiation-inducing properties of β-car^{ox} and some of its fractions, it is instructive not only to compare the effects obtained with those of the better known *all-trans* retinoic acid, which is recognized as being able to regulate differentiation capacities of several mammalian cell types, but to determine also the effect upon cells when β-car^{ox} and retinoic acid are used together. Table 12 presents qualitative data illustrating the results obtained with various combinations of β-car^{ox} and retinoic acid in both the NB4 and IMR32 cell lines.

When an equipotent concentration of β-car^{ox} is combined with retinoic acid, their differentiation-inducing effects cancel out. However, when the concentration of either one of them is increased relative to the other, differentiation-induction is restored. Surprisingly, when the concentration of both agents together is raised, significant enhancement of differentiation is observed.

Many differentiation markers are expressed transiently during the differentiation program. In the NB4 cell line, protein CD33 is associated with early stages of differentiation, followed by protein CD11b, an intermediate marker, and eventually by protein CD15, an advanced differentiation marker. Enhanced differentiation therefore will be characterized by a decrease in the expression and intensity of the CD33 marker and an increase in expression and intensity of the CD15 marker.

**Table 12.**

| Differentiating properties of mixtures of oxidized β-car^{ox} and retinoic acid. | | |
|---|---|---|
| Retinoic Acid | β-Car^{ox} | Differentiation |
| L | - | yes |
| - | L | yes |
| L | L | no |
| H | L | yes |
| L | H | yes |
| H | H | yes |
| | | (enhanced) |
| Note: The letters L and H indicate low and high concentration, respectively. A dash indicates the absence of the indicated agent. | | |

Table 13 presents quantitative data on the effect of all-trans retinoic acid, β-car^{ox} and some of its fractions on the level of expression of these specific differentiation markers in NB4 cells, supplemented either with individual fractions or various binary combinations with all-trans retinoic acid. The results indicate that β-car^{ox} is not as effective as retinoic acid (β-car^{ox} requires more time to fully express its effect). Combined treatment with retinoic acid and β-car^{ox}, or some of its fractions, results in inhibition of differentiation (an antagonistic effect) as has been already noted above for β-car^{ox} (Table 12), but with fraction IG2 differentiation proceeds further than with either IG2 or retinoic acid alone (a synergistic effect).

This finding that β-car^{ox} and some of its fractions can both interfere with and enhance the action of retinoic acid implies that, because retinoic acid operates through and interacts with the superfamily of nuclear receptors (including the retinoid, thyroid hormone, vitamin D₃ and orphan receptors), β-car^{ox} and some of its fractions are specifically targeting this family of receptors and affecting the cell at the level of nuclear DNA transcription.

The enhancement of differentiation obtained under certain conditions indicated above offers the prospect that an appropriate combination of retinoic acid and β-car^{ox} or the fraction IG2 will be a more effective therapy than retinoic acid alone. There is particular relevance to this because retinoic acid is shortly to become generally available for the treatment of acute promyleocytic leukemia (APL), despite the fact that it provides only 5-6 months of remission before it becomes ineffective in treating the resurgent cancer. Our results support the possibility that a combination of two agents, particularly those indicated above, will push leukemic cells more fully down a terminal differentiation path, increasing the likelihood that their return to a proliferative state is blocked.

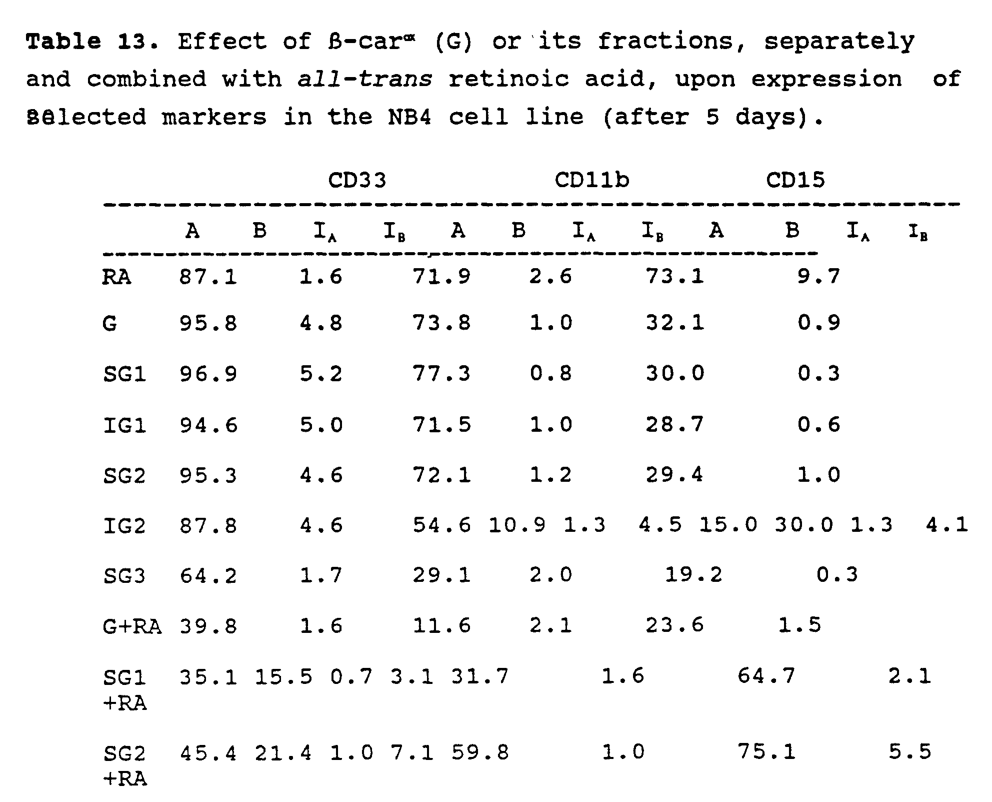

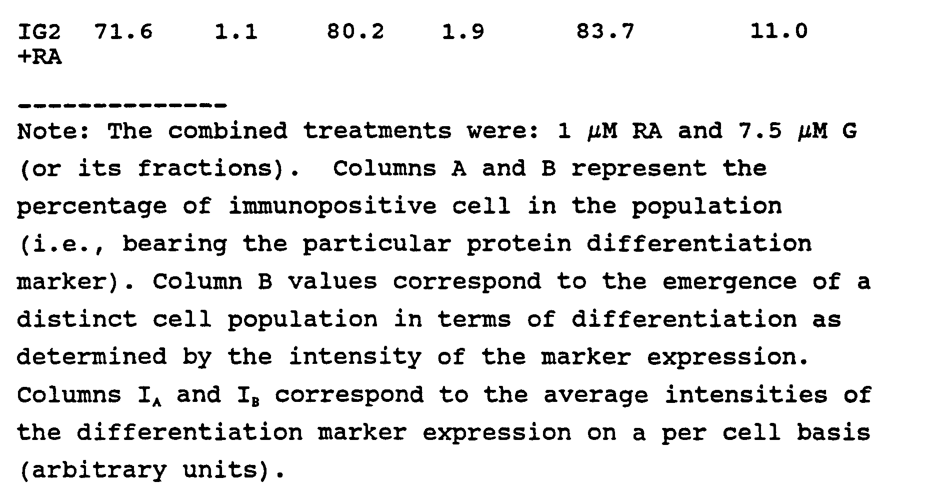

The hypothesis that β-car^{ox} is affecting the cell at the level of nuclear DNA transcription through the interactions with the above mentioned family of receptors is further supported by the results of combined treatment of another cell line, IMR32, with retinoic acid and β-car^{ox}. As in the case of NB4, antagonistic or synergistic effects(depending on the concentrations of both agents) can be observed (see Table 7a). For example, the expression of neuorofilament, NF (a marker indicating that the cells differentiated into neurons), is maximized at 1 *µ*M RA and 5 *µ*M β-car^{ox} when each is used alone. When used together at these concentrations they cancel each other out and the expression of NF is similar to that of the control (antagonism). However, when the concentration of RA is increased to 2.5 *µ*M and the concentration of β-car^{ox} is Seduced to 3 *µ*M, the expression of NF is higher then for either of them alone (synergy). Expression of glial fibrilary acidic protein, GFAP (a marker indicating differentiation towards glial cells), which is enhanced by both agents is as well subject to complex interaction. For example, whereas retinoic acid itself is most effective at low concentration of 1 *µ*M, the presence of β-car^{ox} increases this optimal concentration up to 10 *µ*M depending on the concentration of β-car^{ox} (see table 13a).

**Table 13a.**

| Effect of β-car^{ox} (G), separately and combined with *all-trans* retinoic acid, upon expression of selected markers in the IMR32 cell line (after 48 h). | | | | | |
|---|---|---|---|---|---|
| RA [µM] | β-car^{ox} [µM] | NF | | GFAP | |
| | | % | intensity | % | intensity |
| 0 | 0 | 96.0 | 4.3 | 65.0 | 1.7 |
| 1 | 0 | 96.4 | 14.2 | 94.4 | 18.4 |
| 2.5 | 0 | 97.2 | 11.9 | 90.5 | 6.1 |
| 5 | 0 | 95.5 | 8.4 | 87.5 | 5.2 |
| 10 | 0 | 92.0 | 7.9 | 83.1 | 4.0 |
| 0 | 3 | 90.6 | 7.7 | 91.5 | 5.2 |
| 0 | 5 | 98.7 | 53.0 | 87.0 | 5.2 |
| 0 | 7.5 | 96.1 | 8.6 | 94.2 | 8.7 |
| 0 | 10 | 95.8 | 8.5 | 88.1 | 4.5 |
| 1 | 5 | 94.1 | 6.4 | 98.2 | 22.4 |
| 1 | 7.5 | 94.7 | 7.5 | 87.4 | 5.6 |
| 1 | 10 | 98.2 | 17.6 | 89.7 | 6.2 |
| 2.5 | 3 | 98.3 | 70.0 | 87.7 | 5.6 |
| 2.5 | 5 | 94.7 | 7.2 | 97.9 | 21.7 |
| 2.5 | 7.5 | 98.7 | 22.2 | 92.7 | 10.4 |
| 2.5 | 10 | 93.0 | 6.1 | 97.6 | 20.4 |
| 5 | 3 | 97.5 | 17.0 | 79.1 | 3.4 |
| 5 | 5 | 94.7 | 7.1 | 90.3 | 4.8 |
| 5 | 7.5 | 95.3 | 9.1 | 96.8 | 18.4 |
| 5 | 10 | 96.7 | 15.0 | 97.4 | 18.5 |
| 10 | 3 | 93.4 | 6.6 | 89.6 | 5.3 |
| 10 | 5 | 94.5 | 7.7 | 89.5 | 7.0 |
| 10 | 7.5 | 90.6 | 7.7 | 93.3 | 7.9 |
| 10 | 10 | 95.1 | 7.0 | 97.0 | 15.9 |

### Effect of β-car^{ox} upon the cell cycle

The distribution of the β-car^{ox}-treated cell population among the three phases of the cell cycle (G1 phase (resting cells), S phase DNA synthesis stage), and G2 phase (chromosome doubling)) shows an accumulation of cells in the S phase. There is no G1 block. This effect is due to a lengthening of the duration of the cell cycle. It was found that the β-car^{ox}-treated cells completed a cell cycle in 36 h whereas the duration of the cell cycle in controls and retinoic acid-treated matching samples was 22 and 18 h, respectively. The data were acquired using flow cytometry analysis of the individual intracellular DNA content of cells from synchronized populations. Sampling was done every 5 h over a period of 48 h. Two types of cells were used: growing in suspension (NB4 leukemia cells) and growing as a adherent cells (L6 myoblasts).

### Other biological properties.

Effect of β-car^{ox} in decreasing the level of cellular glutathione and on increasing the sensitivity of a cancer cell line towards a conventional chemotherapeutic agent. Often, classical anticancer drugs are ineffective because cancerous cells acquire resistance towards the treatment. In many cases, this phenomenon is linked to an increased level of glutathione (GSH), which protects cancerous cells by reacting with the active component of the drug and thereby assisting its removal from the cell. Data given in Table 14 show that β-car^{ox} is able to substantially lower the level of GSH in the DA-3 mouse mammary cancer cell line, which is the same cell line used in the animal model for assessing antitumor activity. The results in Table 14 compare very favorably with the corresponding effect of buthionine sulfoximine (BSO), a more toxic inhibitor of GSH biosynthesis, which currently is undergoing testing as an anticancer agent.

**Table 14.**

| Effect of **β**-car^{ox} on the level of glutathione in the DA-3 mouse mammary cancer cell line. | |
|---|---|
| β-car^{ox} [*µ*M] | GSH [nmol/mg protein] |
| 0 | 30 |
| 5 | 38 |
| 10 | 6 |
| 20 | 3 |

The benefit of the cellular GSH-lowering effect is illustrated by what happens to the viability of DA-3 cells (using the MTT test) treated with both β-car^{ox} and melphalan, a classical anticancer agent. The data in Table 15 show that the IC₅₀ of melphalan is reduced dramatically in the presence of low concentrations of β-car^{ox}. (These results are illustrated graphically in Figures 23a and 23b).

This effect suggests that co-administration of β-car^{ox} with a conventional anticancer agent, such as melphalan, will increase the sensitivity of cancerous cells towards the anticancer drug. This can be helpful in one of two ways: a) the same dose can achieve a higher concentration of the drug in the cancerous cells; b) a lower dose will be required for a therapeutic effect thereby reducing the unwelcome side effects resulting from the high, general toxicity of melphalan.

**Table 15.**

| Effect of oxidized β-carotene on IC₅₀ of melphalan in the DA-3 mouse mammary cancer cell line model as estimated by MTT test. | |
|---|---|
| β-Car^{ox} (*µ*M) | IC₅₀ of melphalan (*µ*M) |
| 0.0 | 100 |
| 5.0 | 40 |
| 7.5 | 44 |
| 10.0 | 6.6 |
| 20.0 | 6.8 |

### Toxicity and Antitumor Activity of β-Car^{ox} In Vivo

### Antitumor effect of β-car^{ox} in other animal models. Nude mice xenografts

Evaluation of the antitumor activity of β-car^{ox} has been extended to include a study of the effect upon tumor growth arising from the transplantation of A2780 human ovarian cancer cells into nude mice. Figure 24 shows that intraperitoneal administration of β-car^{ox} significantly inhibits the growth of this considerably more aggressive tumor (as compared to the DA-3 model).

### Antitumor effect of β-car^{ox} fractions and related substances

The mouse D1-DMBA-3 (DA-3) mammary adenocarcinoma model was used. The results displayed in Figs. 25a and 25b show that several fractions were at least as potent (fractions SG, F1, F2) or more potent (fractions F1.1, F1.2, F1.3; note lower concentrations used) than β-car^{ox}. The potency of the ketoaldehyde (compound 1, Figure 8) is striking.

Table 16 shows the estimated tumor volume expressed relative to the control (i.e., the group receiving the vehicle only) on day 28 of the experiment. In contrast to what was previously observed for β-car^{ox}, it can be seen that at least in the case of fraction F1 a dose-response effect exists.

**Table 16.**

| In vivo antitumor activity of β-car^{ox} and its fractions in the DA-3 model. | | |
|---|---|---|
| Sample | Dose [mg/kg body] | Relative Tumor Size (28 days) |
| β-car^{ox} | 25 | 0.36 |
| | | |
| SG1 | 25 | 0.30 |
| | | |
| LSG | 25 | 0.49 |
| | | |
| F1 | 5 | 0.72 |
| | 10 | 0.49 |
| | 25 | 0.37 |
| | | |
| F2 | 25 | 0.33 |
| | | |
| F1.1 | 10 | 0.30 |
| F1.2 | 10 | 0.47 |
| F1.3 | 10 | 0.47 |
| | | |
| Ketoaldehyde | 10 | 0.44 |
| 20% Ethanol | N/A | (1.00) |

An alternative delivery mode was tested on the same model for two fractions, F1 and F2. Data in table 17 show that both fractions when applied orally (p.o.) are at least as effective as when applied intraperitoneally (i.p.).

**Table 17.**

| Comparison of in vivo antitumor activity of fractions F1 and F2 delivered i.p and p.o. in the DA-3 model. | |
|---|---|
| Sample at 25 mg/kg body | Relative Tumor Size (21 days) |
| F2 p.o. | 0.50 |
| F2 i.p. | 0.48 |
| F1 p.o. | 0.34 |
| F1 i.p. | 0.43 |
| 20% Ethanol | (1.00) |

## Claims

1. Use of a product for the manufacture of a medicament for use in treating a tumour or skin cell proliferation in a human or animal, wherein the product is a mixture containing more than one oxidised compound, obtainable by the process of oxidation of a carotenoid compound with oxygen, such that 6 to 8 molecules of oxygen react per molecule of the carotenoid compound.

2. Use according to claim 1, wherein the process also comprises fractionation of the oxidised mixture, according to molecular weight.

3. Use according to claim 2, wherein the fractionation is effected by solvent precipitation.

4. Use according to claim 2, wherein the fractionation is effected by size exclusion chromatography.

5. Use according to any of claims 2 to 4, wherein the process comprises further fractionation or isolation, according to polarity.

6. Use according to any preceding claim, wherein the oxidation is conducted in an organic solvent.

7. Use according to any of claims 1 to 5, wherein the oxidation is conducted on said compound in the solid state.

8. Use according to any preceding claim, wherein said compound is β-carotene, lycopene or canthaxanthin.

9. Use according to any preceding claim, wherein said compound is β-carotene.

10. Use according to claim 8, wherein said compound is lycopene.

11. Use according to any preceding claim, wherein the oxidation is conducted in the absence of catalysts, initiators or inhibitors of oxidation.

12. Use according to any preceding claim, wherein the mixture contains a polymeric or oligomeric component.

13. Use according to any of claims 1 and 9 to 12, wherein each oxidised compound has a molecular weight of less than 700 Da.

14. Use according to claim 13, wherein each oxidised compound has a molecular weight of less than 300 Da.

15. Use according to any preceding claim, wherein the animal or human is also treated with a conventional anti-cancer agent.

16. Use of 2-methyl-6-oxo-2,4-heptadienal, for the manufacture of a medicament for use in treating a tumour in an animal or human.

## Patentansprüche

1. Verwendung eines Produkts zur Herstellung eines Medikaments zur Verwendung bei der Behandlung eines Tumors oder von Hautzellproliferation bei einem Menschen oder Tier, wobei das Produkt eine mehr als eine oxidierte Verbindung enthaltende Mischung ist, erhältlich durch das Verfahren der Oxidation einer Carotinoid-Verbindung mit Sauerstoff, so daß 6 bis 8 Moleküle Sauerstoff pro Molekül der Carotinoid-Verbindung reagieren.

2. Verwendung nach Anspruch 1, worin das Verfahren auch die Fraktionierung der oxidierten Mischung nach dem Molekulargewicht umfaßt.

3. Verwendung nach Anspruch 2, worin die Fraktionierung durch Präzipitation in einem Lösungsmittel bewirkt wird.

4. Verwendung nach Anspruch 2, worin die Fraktionierung durch Größenausschlußchromatographie bewirkt wird.

5. Verwendung nach irgendeinem der Ansprüche 2 bis 4, worin das Verfahren ferner die Fraktionierung oder Isolierung nach der Polarität umfaßt.

6. Verwendung nach irgendeinem der vorhergehenden Ansprüche, worin die Oxidation in einem organischen Lösungsmittel durchgeführt wird.

7. Verwendung nach irgendeinem der Ansprüche 1 bis 5, worin die Oxidation mit der Verbindung in festem Zustand durchgeführt wird.

8. Verwendung nach irgendeinem der vorhergehenden Ansprüche, worin die Verbindung β-Carotin, Lycopin oder Canthaxanthin ist.

9. Verwendung nach irgendeinem der vorhergehenden Ansprüche, worin die Verbindung β-Carotin ist.

10. Verwendung nach Anspruch 8, worin die Verbindung Lycopin ist.

11. Verwendung nach irgendeinem der vorhergehenden Ansprüche, worin die Oxidation in Abwesenheit von Oxidations-Katalysatoren, -Initiatoren oder -Inhibitoren durchgeführt wird.

12. Verwendung nach irgendeinem der vorhergehenden Ansprüche, worin die Mischung eine polymere oder oligomere Komponente enthält.

13. Verwendung nach irgendeinem der Ansprüche 1 und 9 bis 12, worin jede oxidierte Verbindung ein Molekulargewicht von weniger als 700 Dalton aufweist.

14. Verwendung nach Anspruch 13, worin jede oxidierte Verbindung ein Molekulargewicht von weniger als 300 Dalton aufweist.

15. Verwendung nach irgendeinem der vorhergehenden Ansprüche, worin das Tier oder der Mensch auch mit einem herkömmlichen Mittel gegen Krebs behandelt wird.

16. Verwendung von 2-Methyl-6-oxo-2,4-heptadienal zur Herstellung eines Medikaments zur Verwendung bei der Behandlung eines Tumors bei einem Tier oder Menschen.

## Revendications

1. Utilisation d'un produit pour la fabrication d'un médicament à utiliser dans le traitement d'une tumeur ou d'une prolifération de cellules de la peau chez l'homme ou l'animal, dans laquelle le produit est un mélange contenant plus d'un composé oxydé, que l'on peut obtenir par un procédé d'oxydation d'un composé caroténoïde par l'oxygène, tel que 6 à 8 molécules d'oxygène réagissent par molécule du composé caroténoïde.

2. Utilisation selon la revendication 1, dans laquelle le procédé comprend aussi le fractionnement du mélange oxydé, suivant le poids moléculaire.

3. Utilisation selon la revendication 2, dans laquelle le fractionnement est effectué par précipitation à l'aide d'un solvant.

4. Utilisation selon la revendication 2, dans laquelle le fractionnement est effectué par chromatographie à exclusion de taille.

5. Utilisation selon l'une quelconque des revendications 2 à 4, dans laquelle le procédé comprend de plus le fractionnement ou l'isolement, selon la polarité.

6. Utilisation selon l'une quelconque des revendications précédentes, dans laquelle l'oxydation est conduite dans un solvant organique.

7. Utilisation selon l'une quelconque des revendications 1 à 5, dans laquelle l'oxydation est conduite sur ledit composé à l'état solide.

8. Utilisation selon l'une quelconque des revendications précédentes, dans laquelle ledit composé est du β-carotène, du lycopène ou de la canthaxanthine.

9. Utilisation selon l'une quelconque des revendications précédentes, dans laquelle ledit composé est du β-carotène.

10. Utilisation selon la revendication 8, dans laquelle ledit composé est du lycopène.

11. Utilisation selon l'une quelconque des revendications précédentes, dans laquelle l'oxydation est conduite en l'absence de catalyseurs, d'initiateurs ou d'inhibiteurs d'oxydation.

12. Utilisation selon l'une quelconque des revendications précédentes, dans laquelle le mélange contient un composant polymérique ou oligomérique.

13. Utilisation selon l'une quelconque des revendications 1 et 9 à 12, dans laquelle chaque composé oxydé a un poids moléculaire inférieur à 700 Da.

14. Utilisation suivant la revendication 13, dans laquelle chaque composé oxydé a un poids moléculaire inférieur à 300 Da.

15. Utilisation selon l'une quelconque des revendications précédentes, dans laquelle l'animal ou l'homme est également traité à l'aide d'un agent anticancéreux classique.

16. Utilisation du 2-méthyl-6-oxo-2,4-heptadiénal pour la fabrication d'un médicament à utiliser dans le traitement d'une tumeur chez l'animal ou chez l'homme.
